(19) Europäisches Patentamt
European Patent Office
Office européen des brevets

(11) EP 1 914 309 A1

# (12) EUROPEAN PATENT APPLICATION

(43) Date of publication:
**23.04.2008 Bulletin 2008/17**

(51) Int Cl.:
***C12N 15/10*** *(2006.01)* ***C12N 15/86*** *(2006.01)*

(21) Application number: **06291637.4**

(22) Date of filing: **20.10.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicants:
- **Eurofins Viralliance Inc.**
  **Memphis TN 38119 (US)**
- **INSTITUT NATIONAL DE LA SANTE ET DE LA RECHERCHE MEDICALE (INSERM)**
  **75013 Paris (FR)**

(72) Inventors:
- **Skrabal, Katharina**
  **75015 Paris (FR)**
- **Lebel-Binay, Sophie**
  **94800 Villejuif (FR)**
- **Roulet, Vanessa**
  **75017 Paris (FR)**

(74) Representative: **Colombet, Alain André et al**
**Cabinet Lavoix,**
**2, Place d'Estienne d'Orves**
**75441 Paris Cedex 09 (FR)**

## (54) Method of assaying HIV-1 tropism for CCR5 and/or CXCR4 co-receptor and/or susceptibility to entry inhibitors

(57) The invention relates to a method of characterising the cellular tropism of Human Immunodeficiency Virus type 1 (HIV-1) viruses, i.e. the capacity of HIV-1 viruses to use the CXCR4 and/or CCR5 receptors for cell entry. The invention also relates to a method of assessing susceptibility or resistance of HIV-1 viruses to entry inhibitors, in particular those targeting HIV-1 co-receptors.

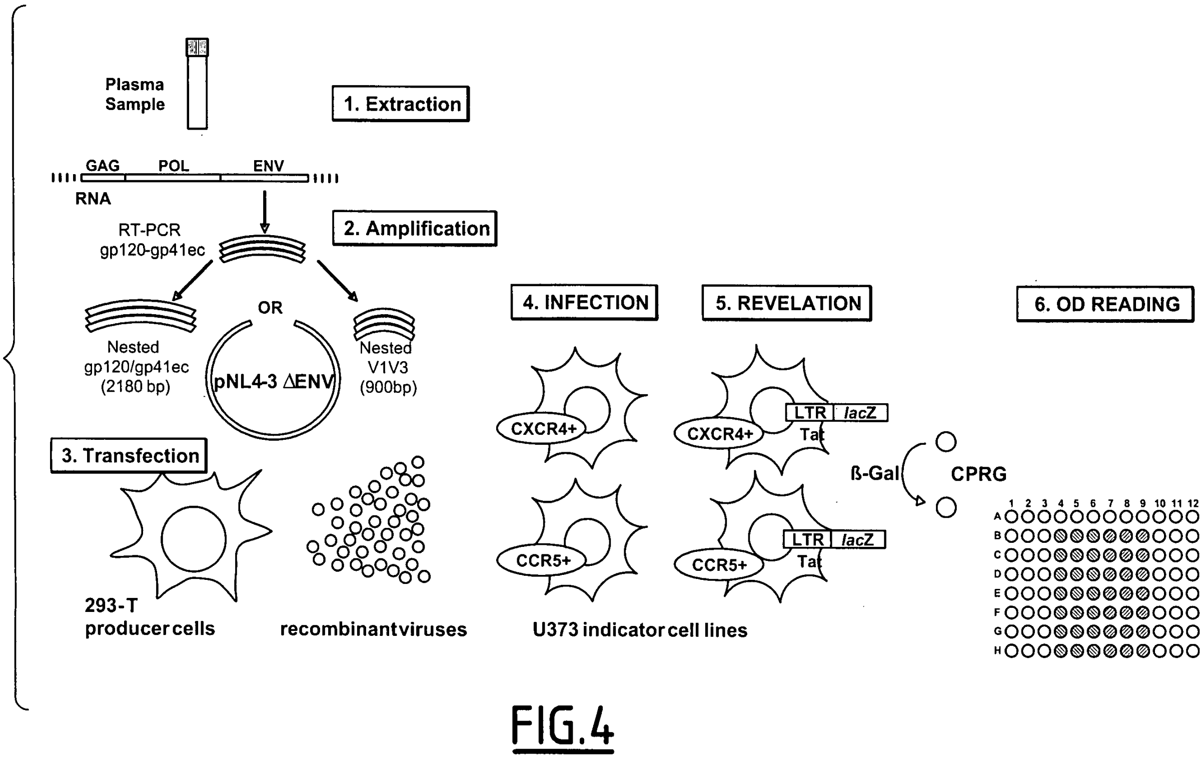


FIG.4

EP 1 914 309 A1

## Description

The invention relates to a method of characterising the cellular tropism of Human Immunodeficiency Virus type 1 (HIV-1), i.e. the capacity of HIV-1 virus to use the CXCR4 and/or CCR5 receptors for cell entry. The invention also relates to a method of assessing susceptibility of resistance of HIV-1 viruses to entry inhibitors, in particular those targeting HIV-1 co-receptors.

The entry of HIV into cell is mediated by several proteins, including the viral envelope proteins gp41 and gp120. Both are derived from the precursor protein gp160 and remain non-covalently bound after the cleavage of gp160. Gp41 spans the viral membrane with endo- and ecto-domains placed inside and outside of the viral membrane region, respectively. The ectodomain is critical to interactions of gp41 with gp120 and ultimately to virus-cell fusion.

The initial step of HIV replication cycle involves attachment of the virion to helper T cells through the interactions of HIV-gp120 glycoprotein with cellular protein CD4. However, HIV-1 requires interaction with CD4 and a co-receptor to trigger fusion of viral and cellular membranes and entry into cells. The most important secondary receptors *in vivo* are the chemokine receptors CCR5 and CXCR4. Over the course of the infection, the co-receptor usage of HIV-1 changes from a preference for CCR5 to a preference for CXCR4 in about 50% of infected individuals. The switch from CCR5-using to CXCR4-using HIV-1 variants is associated with accelerated CD4+ T-cell decline and progression to AIDS. The phenotypic switch has been associated with mutations in the V3 region of gp120 envelope glycoprotein.

In the standard terminology for HIV-1 phenotypes, CCR5-using variants are referred as R5 viruses, CXCR4-using variants are referred as X4 viruses, and variants that are able to use both co-receptors are referred as R5X4 viruses.

Methods of analysing phenotypic characteristics of HIV viruses, including cellular tropism and resistance/susceptibility to cell entry inhibitors, have been described in the art.

For instance Kootstra and Schuitemaker (2005, Methods Mol Biol., 304:317-25) describe that cellular tropism of HIV-1 tropism can be determined on primary monocyte derived macrophages, established T-cell lines and promonocytic cell lines.

Peters et al. (2006, J. Virol., 80 (3), 6324-6332) disclose determination of tropism using a NL4-3 vector deleted of the envelope region, used for cotransfection of 293T producer cells with a plasmid pSVIII-Env+ (carrying PCR-amplified patients' envelopes). Transfected 293T cells are used in a cell fusion assay with GHOST cells expressing CCR5 or CXCR5 co-receptors and syncytia formation is observed by light microscopy.

In Gray et al. (2005, Virology, 337(2), 384-98), primary virus is isolated from patients' PBMCs and used for infection of Cf2-Luc cells transfected with CD4 and CCR5 or CXCR4. Infection is determined after cell lysis using a luciferase assay.

The article Kulkarni et al. (2005, Virology, 337(1), 68-75) describes a GHOST cell assay, with indicator cells harbouring GFP under control of HIV-2 Tat. Infection is observed using FACS analysis on infected cells.

The international patent application WO 02/27321 describes a diagnostic method to determine whether CXCR4 or CCR5 strains are present in a patient infected with HIV, which method relies on a cell fusion assay between cells transformed with an HIV envelope gene variant cloned from an infected patient, and an indicator cell line expressing an HIV envelope-compatible co-receptor. The indicator cell line contains an inducible reporter gene construct which is activated upon fusion with a cell containing a suitable transcriptional activator and/or transcription factor.

The international patent application WO 02/38792 describes a Recombinant Virus Assay (RVA) for phenotypic characterisation of HIV, including determination of HIV tropism and resistance/susceptibility to cell entry inhibitors. The method comprises PCR amplification of a region of the viral genome liable to comprise a mutation, and the transfection of a first host cell with said amplified region together with a vector comprising the parts of HIV genome required for viral replication except for the amplified region, in order to obtain a chimeric virus by homologous recombination. The method then involves the production of viral particles by the first host cell and the infection of a second host cell with the viral particles. The second host cell is chosen so as to be liable to be infected by HIV virus or HIV pseudotyped virus and comprises a marker gene which is activated upon viral infection. According to an embodiment, the amplified region consisted of the majority of the gp120 sub-unit and the extracellular portion (ectodomain) of the gp41 sub-unit. According to another embodiment, the amplified region consisted of the domains ranging from the V1 loop to the V3 loop of HIV-1.

However, improvements had still to be done in order to increase sensitivity of the method for HIV-1 subtype non-B viruses, and to achieve more accurate and easier analysis of tropism.

More recently, the international patent application WO 03/070985 has described a co-receptor tropism assay which comprises transfecting a first host cell with two different vectors, one containing the cloned envelope of a patient's sample, the other one lacking a sequence encoding the envelope protein and carrying a gene producing a detectable signal; culturing the transfected host cell to produce viral particles; infecting a second host cell with the viral particles; and measuring the detectable signal. In particular, a co-receptor tropism screening assay is described
wherein the second host cell is a cell line expressing either both CD4 and CCR5 or both CD4 and CXCR4. The assay is optionally performed in the presence of a drug inhibiting either R5 or X4 tropic viruses.

The phenotypic tropism co-receptor assays described in the international patent applications WO 02/27321 and WO 03/070985 have been compared and were shown to give overall 85% of concordant results ("Is there a Gold Standard for Determining HIV Coreceptor Usage in Clinical Samples? A Comparison of two Phenotypic Tropism Assays and a Bioinformatic Model" A. Low, K. Skrabal, W. Dong, F. Mammano, T. Sing, P. Cheung and P.R. Harrigan, 13th Conference on Retroviruses and Opportunistic Infections, Denver, February 5-8, 2006).

However, there is still a need for a tropism test with higher sensibility for the detection of X4-tropic viruses, especially in mixtures of viruses harbouring a minority of viruses using CXCR4 in addition to or in the place of CCR5, where previous tests were unable to detect these quasispecies. Additionally the test should allow the determination of tropism of subtype B and non-B samples with high specificity and sensitivity, the increasing prevalence of subtype non-B samples and circular recombinant forms being a major problem for analysis.

This goal is achieved by the present invention which provides a RVA assay enabling to test viral tropism with high sensitivity. The method of the invention tests HIV-1 group M, subtype B and non-B samples, using two different amplification cassettes: Env (gp120/gp41ec) which has the advantage to allow in parallel the determination of susceptibility of a virus to entry/fusion/co-receptor inhibitors, and V1V3 which may be more convenient for the amplification of non-B samples, when either PCR or RVA were not successful. More specifically, it is proposed to perform a first amplification using the Env cassette, followed - in case of a negative PCR or RVA result - by a second amplification attempt using the V1V3 cassette. The addition of CCR5- and CXCR4-inhibitors allows the increase of the sensitivity of the assay when values at the detection limit did not allow a reliable interpretation in previous tests. Thus, the precise analysis of OD ratios in presence and absence of inhibitors proves (> 5%) the usage of CCR5- and/or CXCR4-coreceptors for viral entry.

*Definitions*

"HIV-1" denotes the human immunodeficiency virus type 1. Phylogenetic analysis of many isolates of HIV-1 from Africa and from other regions of the world have revealed three major lineages of HIV-1: viruses of group M (for main) are responsible for the majority of infections worldwide, group O (for outlier) is a relatively rare group currently found in Cameroon, Gabon, and France, and group N gathers non-M/non-O viruses. Within group M, in addition to subtype B, at least eight distinct non-B subtypes (A through H) and circulating recombinant forms (CRFs) have been proposed.

In the instant application, nucleotide positions are indicated by reference to the genomic sequence of HIV-1 (p)NL4-3 (Genebank accession number U26942). Nucleotides 6171 to 9090 of (p)NL4-3 are shown on Figures 2 to 4.

The "pNL4-3" plasmid is the most widely used vector for *in vitro* manipulations of the HIV-1 proviral sequences. pNL4-3 is a recombinant (infectious) proviral clone that contains DNA from HIV isolates NY5 (5' half) and BRU (3' half). The site of recombination is the *EcoRI* site at positions 5743-5748. The construction of pNL4-3 has been described in Adachi et al. (1986, J. Virol., 59 (2), 284-291).

"Primers" which are used for PCR amplification are oligonucleotides, i.e. nucleic acids generally of at least 12, preferably at least 15, and more preferably at least 20 nucleotides, and preferably no more than 30, more preferably no more than 40, still preferably no more than 50 nucleotides, which are hybridisable under high stringency conditions, and preferably complementary, to a region of double stranded HIV-1 cDNA molecule. Advantageously, the sequence of a primer is designed so that the primer binds to a region of double stranded HIV-1 cDNA molecule which is highly conserved among strains of HIV-1 group M, subtype B or non-B. High stringency hybridization conditions typically correspond to the highest Tm, e.g., 50 % formamide, 5x or 6x SCC where SCC is a 0.15 M NaCl, 0.015 M Na-citrate. Appropriate conditions of temperature and solution ionic strength may be selected by the one skilled in the art (see Molecular Cloning, A Laboratory Manual Joseph Sambrook and David W Russell, Cold Spring Harbor Laboratory Press, New York).

In the context of the invention, an "entry inhibitor" denotes a molecule, for instance a protein-based, a peptide-based, a peptide-mimetic, a synthetic peptide analog or a non peptide molecule (e.g. a small molecule), which inhibits entry of HIV-1 into cells by inhibiting either (i) interaction between gp120 and CD4, or (ii) interaction between CD4 and a co-receptor ("co-receptor inhibitors"), or (iii) fusion of viral and cellular membranes ("fusion inhibitor").

As used herein, a "CCR5 co-receptor inhibitor" denotes a molecule which prevents utilisation of CCR5 in the process of HIV infection. A CCR5 co-receptor inhibitor may typically act by interfering with gp120 binding to CCR5 co-receptor, or by inducing CCR5 internalisation thereby removing the co-receptor from the membrane surface. Examples of CCR5 co-receptor inhibitors include, without limitation, macrophage inflammatory protein MIP-1$\alpha$ and MIP-1$\beta$, RANTES (Alkhatib et al., 1996, Science; 272: 1955-1958), or synthetic an analogs thereof (AOP-RANTES (Simmons et al., 1997, Science; 276: 276-279), PSC-RANTES (Hartley et al., 2004, Proc Natl Acad Sci U S A; 101: 16460-16465)), LD78b, MIP-1$\alpha$ isoform (Menten et al., 1999, J Clin Invest; 104: R1-R5), Sch-C (Palani et al., 2001, J Med Chem., 44 (21):3339-42.), Sch-D (Vicriviroc), UK-427, 857 (Maraviroc), GSK-873140 (Aplaviroc), TAK-779 (CAS registry number: 229005-80-5), and anti-CCR5 antibodies. In particular anti-CCR5 monoclonal antibodies may be used, such as antibodies 3A9, SD7, 45502.111, 45549.111, 45531.111 and 45523.111 (http://www.aidsreagent.org).

As used herein, a "CXCR4 co-receptor inhibitor" denotes a molecule, which prevents utilisation of CXCR4 in the process of HIV infection. A CXCR4 co-receptor inhibitor may typically act by interfering with gp120 binding to CXCR4 co-receptor, or by inducing CXCR4 internalisation thereby removing the co-receptor from the membrane surface. Examples of CXCR4 co-receptor inhibitors include, without limitation, Stromal cell-derived factor-1$\alpha$ (SDF-1$\alpha$) and SDF-1$\beta$, N-$\alpha$-acetyl-nona-D-arginine amide (ALX-40-4C), synthetic polyphemusins (T22) or analogs thereof (T140) (Kazmierski et al., 2006, Chemical Biology & Drug Design, 67:13), KRH1636 (Ichiyama et al., 2003, Proc Nat Acad Sci U S A, 100: 4185-4190), AMD3100 (De Clercq, 2003, Nat Rev Drug Discov;2: 581-587), and anti-CXCR4 antibodies, such as 12G5, 44717.111, 44708.111, and 44716.111 (http://www.aidsreagent.org).

Examples of fusion inhibitors include T20 (also known as fuzeon or enfuvirtide) and T-1249.

A "saturating dose of an inhibitor" denotes a concentration of inhibitor at which cell infection by a virus having tropism for said cell is 100% blocked by the inhibitor, which is function of the inhibitor. For example, up to 2 $\mu$g/ml for AMD3100 inhibitor, more than 250 ng/ml for RANTES and more than 12 $\mu$g/ml for T20.

By "R5-tropic virus" is meant a HIV-1 virus which uses CCR5 co-receptor for entry into cells.

By "X4-tropic virus" is meant a HIV-1 virus which uses CXCR4 co-receptor for entry into cells.

By "R5/X4-tropic virus" is meant a HIV-1 virus which may use any of CCR5 and CXCR4 co-receptors for entry into cells.

The term "U373-CCR5" denotes U373MG-CD4 cells stably transfected with the *Escherichia coli* $\beta$-galactosidase gene (*lacZ*) under transcriptional control of the HIV-1 long terminal repeat (LTR) which express the CCR5 co-receptor (Labrosse et al., 1998, J Virol., 72(8): 6381-6388).

The term "U373-CXCR4" denotes U373MG-CD4 cells stably transfected with the *Escherichia coli* $\beta$-galactosidase gene (*lacZ*) under transcriptional control of the HIV-1 long terminal repeat (LTR) which express the CXCR4 co-receptor (Labrosse et al., 1998, J Virol., 72(8): 6381-6388).

*Recombinant Virus Assay of R5 and/or X4 tropism*

The invention provides a Recombinant Virus Assay (RVA) of tropism which allows the evaluation of viral tropism (i.e. the utilisation of co-receptors CCR5 and/or CXCR4 in addition to CD4 for entry into target cells) of HIV-1 group M viruses, subtype B and non-B, as well as CRF variants. To that end, after extraction of viral RNA from patients' plasma samples, a specific region of the viral envelope (gp120 and the ectodomain of gp41 - "gp41ec") is amplified using an RT-PCR. The gp120-gp41 ec region, and optionally the region spanning the tropism determinant region V1-V3, is then amplified using a Nested PCR. Recombinant viruses, comprising the HIV envelope isolated from patients' samples, are produced by co-transfection of 293T producer cells using the PCR-product and the plasmid pNL4-3 deleted of the corresponding region in the envelope (either pNL43$\Delta$env or pNL4-3$\Delta$V). Viral supernatants are used to infect U373MG-CD4 indicator cells expressing either the CCR5 or CXCR4 co-receptor (U373-CCR5 or U373-CXCR4), in presence or absence of co-receptor inhibitors at a saturating dose. The indicator cells contain the *lacZ* gene under control of the HIV-1 LTR which allows the determination of viral infection and thus of viral tropism using a colorimetric assay based on the expression of the $\beta$-galactosidase by Tat (see Figure 4).

In the RVA tropism, viral infection is detected independently for both co-receptors. A sample is considered positive when the ratio [OD sample/ OD control] reaches or exceeds 1.5 (the control being an R5-tropic virus on CXCR4+ cells or a X4-tropic virus on CCR5+ cells). The addition of co-receptor inhibitors at saturating dose allows to specifically block the interaction of X4 tropic viruses with the CXCR4 co-receptor or R5 tropic viruses with the CCR5 co-receptor, and thus to specifically inhibit viral entry at 100% via the corresponding co-receptor.

Accordingly, the invention provides a method of assaying tropism of HIV-1 group M viruses present in a biological sample of a patient for CCR5 and/or CXCR4 co-receptor, which method comprises the steps consisting of:

a) subjecting nucleic acids extracted from said biological sample of a patient to a PCR amplification with a pair of primers bordering a region of HIV-1 group M genome coding for all, or essentially all, the gp120 sub-unit and for the ectodomain of gp41 sub-unit;

b) preparing a vector comprising the parts of an HIV-1 group M virus genome required for viral replication except for all or part of the region amplified in step (a);

c) transfecting a producer cell with the amplified nucleic acids of step (a) and the vector prepared in step (b) to obtain a chimeric virus by homologous recombination;

d) culturing said producer cell under conditions enabling the production of viral particles (during a single replication cycle);

e) infecting an indicator cell, which is liable to be infected by an HIV-1 group M virus, which comprises a marker gene which is only activated upon viral infection and which expresses either CCR5 or CXCR4 co-receptor, with the viral particles obtained in step (d),

wherein (e1) said infection is carried out in the presence and in the absence of a saturating dose of a CCR5 co-receptor inhibitor where said indicator cell expresses CCR5, or (e2) said infection is carried out in the presence and in the absence of a saturating dose of a CXCR4 co-receptor inhibitor where said indicator cell expresses CXCR4;
f) separately, (f1) where said indicator cell expresses CCR5, infecting at least another indicator cell of the same type with a X4-tropic control virus in the presence and in the absence of said saturating dose of CCR5 co-receptor inhibitor,
or (f2) where said indicator cell expresses CXCR4, infecting at least another indicator cell of the same type with a R5-tropic control virus in the presence and in the absence of said saturating dose of CXCR4 co-receptor inhibitor;
g) quantifying the level of marker expressed in steps (e1) and (f1), and/or in steps (e2) and (f2); and
h) calculating the ratios

$[sample/control]_{CCR5+}$ = [level of marker expressed in step (e1) / level of marker expressed in step (f1)] in the absence of the CCR5 co-receptor inhibitor, and

$[sample/control]_{CCR5-}$ = [level of marker expressed in step (e1) / level of marker expressed in step (f1)] in the presence of said saturating dose of CCR5 co-receptor inhibitor; and/or

$[sample/control]_{CXCR4+}$ = [level of marker expressed in step (e2) / level of marker expressed in step (f2)] in the absence of the CXCR4 co-receptor inhibitor,

and

$[sample/control]_{CXCR4-}$ = [level of marker expressed in step (e2) / level of marker expressed in step (f2)] in the presence of said saturating dose of CXCR4 co-receptor inhibitor;

wherein a ratio $[sample/control]_{CCR5+}$ equal or above 1.5, while the ratio $[sample/control]_{CCR5}$- is comprised between 0.9 and 1.1 (limits included), is indicative of the presence of HIV-1 group M viruses, in the biological sample from the patient, having tropism for CCR5 co-receptor, and/or
wherein a ratio $[sample/control]_{CXCR4+}$ equal or above 1.5, while the ratio $[sample/control]_{CXCR4-}$ is comprised between 0.9 and 1.1 (limits included), is indicative of the presence of HIV-1 group M viruses, in the biological sample from the patient, having tropism for CXCR4 co-receptor.

As used herein, "a region coding for essentially all the gp120 sub-unit" denotes a sequence consisting of the sequence encoding gp120 (nt 6305-7744) in which no more than 100, preferably 50, more preferably 30, still preferably 15, consecutive nucleotides have been deleted from the 5' end.

The sequence encoding gp41ec consists of nucleotides 7745-8329.

Therefore, the pair of primers bordering a region of HIV-1 group M genome coding for all, or essentially all, the gp120 sub-unit and for the ectodomain of gp41 sub-unit may be selected so that:

- the forward primer hybridises to a region upstream of gp120 coding sequence (for instance a region upstream env sequence), or to a region at the 5' end of gp120 coding sequence, for instance a region upstream of nt 6479 (preferably a region within the 100, preferably 50, nucleotides at the 5' end of gp120 coding sequence); and
- the reverse primer hybridizes to a region downstream of the sequence encoding the ectodomain of gp41 (for instance downstream of position 8264).

For instance, the forward primer may comprise or consist of the sequence E00: TAG AAA GAG CAG AAG ACA GTG GCA ATG A (SEQ ID NO:1; nt 6197-6224), or E10: TTG TGG GTC ACA GTC TAT TAT GGG GT (SEQ ID NO:3; nt 6320-6345), or a fragment thereof. The reverse primer may comprise or consist of the sequence E01-: TCC AGT CCC CCC TTT TCT TTT AAA AA (SEQ ID NO:2; nt 9079-9054), or FuB: GGT GGT AGC TGA AGA GGC ACA GG (SEQ ID NO:4; nt 8521-8500), or a fragment thereof. The said fragments are at least 12, preferably at least 15, and more preferably at least 20 nucleotide-long.

Preferably, the step (a) of PCR amplification of the region coding for gp120 sub-unit and ectodomain of gp41 sub-unit is performed by RT-PCR followed by a nested PCR.

According to an embodiment, said RT-PCR of the extracted nucleic acids uses primers:

E00: TAG AAA GAG CAG AAG ACA GTG GCA ATG A (SEQ ID NO:1), and
E01-: TCC AGT CCC CCC TTT TCT TTT AAA AA (SEQ ID NO:2),
leading to the amplification of a 2882 bp (c)DNA spanning nt 6197-9079, followed by a nested PCR with primers :
E10 : TTG TGG GTC ACA GTC TAT TAT GGG GT (SEQ ID NO:3), and
FuB: GGT GGT AGC TGA AGA GGC ACA GG (SEQ ID NO:4), leading to the amplification of a 2202 bp (nested c)DNA spanning nt 6320-8521 (see Figure 5).

The primers E00, E01- and FuB have been described in the international patent application WO 02/38792.

The suitable cycling conditions RT-PCR and nested PCR may be readily determined by the one skilled in the art. The conditions cycling described in the following examples may be used, advantageously.

The vector used in step (b) is preferably "RVA-Δenv", i.e. a pNL4-3 plasmid, deleted of all or part the gp120-gp41ec region of the envelope gene which has been replaced by a linker containing a suitable restriction site for linearization of the plasmid (i.e. a restriction site which will be found only once in the modified plasmid RVA-Δenv). The region to be deleted in the pNL4-3 plasmid is selected in such a way that the linearised vector contains sequences at its 5' and 3' ends which overlap the amplified nucleic acids, so that homologous recombination can occur between the vector and the amplified nucleic acids. Still preferably, said vector is the pNL4-3 plasmid, deleted of nucleotides 6480-8263 and replaced by a linker containing the *MluI* restriction site.

Where the above method fails to lead to PCR amplification of the extracted nucleic acids and/or if the result of the RVA tropism assay with the gp120-gp41ec cassette is negative, and/or if the biological sample is likely to contain non-B subtypes of HIV-1 group M, the method of amplifying the V1-V3 region of gp120 may be used.

Thus, according to the invention, the method of assaying tropism of HIV-1 group M viruses present in a biological sample of a patient for CCR5 and/or CXCR4 co-receptor may be carried out by implementing the above "gp120-gp41ec" method followed by the "V1V3 method" which is detailed below.

Indeed, the invention also provides a method of assaying tropism of HIV-1 group M viruses present in a biological sample of a patient for CCR5 and/or CXCR4 co-receptor, which method comprises the steps consisting of:

a) subjecting nucleic acids extracted from said biological sample of a patient to a PCR amplification with a pair of primers bordering the entire region of HIV-1 group M genome coding for V1-V3 loops of gp120 sub-unit;
b) preparing a vector comprising the parts of an HIV-1 group M virus genome required for viral replication except for the region coding for V1-V3 loops of gp120 sub-unit;
c) transfecting a producer cell with the amplified nucleic acids of step (a) and the vector prepared in step (b) to obtain a chimeric virus by homologous recombination;
d) culturing said producer cell under conditions enabling the production of viral particles (during a single replication cycle);
e) infecting an indicator cell, which is liable to be infected by an HIV-1 group M virus, which comprises a marker gene which is only activated upon viral infection and which expresses either CCR5 or CXCR4 co-receptor, with the viral particles obtained in step (d),
wherein (e1) said infection is carried out in the presence and in the absence of a saturating dose of CCR5 co-receptor inhibitor where said indicator expresses CCR5, or (e2) said infection is carried out in the presence and in the absence of a saturating dose of CXCR4 co-receptor inhibitor where said indicator cell expresses CXCR4;
f) separately, (f1) where said indicator cell expresses CCR5, infecting at least another indicator cell of the same type with a X4-tropic control virus in the presence and in the absence of said saturating dose of CCR5 co-receptor inhibitor,
or (f2) where said indicator cell expresses CXCR4, infecting at least another indicator cell of the same type with a R5-tropic control virus in the presence and in the absence of said saturating dose of CXCR4 co-receptor inhibitor;
g) quantifying the level of marker expressed in steps (e1) and (f1), and/or in steps (e2) and (f2); and
h) calculating the ratios

$[sample/control]_{CCR5+}$ = [level of marker expressed in step (e1) / level of marker expressed in step (f1)] in the absence of the CCR5 co-receptor inhibitor, and

$[sample/control]_{CCR5-}$ = [level of marker expressed in step (e1) / level of marker expressed in step (f1)] in the presence of the saturating dose of CCR5 co-receptor inhibitor; and/or

$[sample/control]_{CXCR4+}$ = [level of marker expressed in step (e2) / level of marker expressed in step (f2)] in the absence of the CXCR4 co-receptor inhibitor, and

$[sample/control]_{CXCR4-}$ = [level of marker expressed in step (e2) / level of marker expressed in step (f2)] in the presence of the saturating dose of CXCR4 co-receptor inhibitor,

wherein a ratio $[sample/control]_{CCR5+}$ equal or above 1.5, while the ratio $[sample/control]_{CCR5-}$ is comprised between 0.9 and 1.1 (limits included), is indicative of the presence of HIV-1 group M viruses having tropism for CCR5 co-receptor in the biological sample from the patient, and/or
wherein a ratio $[sample/control]_{CXCR4+}$ equal or above 1.5, while the ratio $[sample/control]_{CXCR4-}$ is comprised between 0.9 and 1.1 (limits included), is indicative of the presence of HIV-1 group M viruses having tropism for CXCR4 co-receptor in the biological sample from the patient.

The primers bordering the entire region coding for V1-V3 loops of gp120 sub-unit may be selected so that:

- the forward primer hybridises to a region upstream of the sequence encoding the V1 loop of gp120 coding sequence (for instance a region of gp 120 coding sequence upstream of nucleotide 6612, i.e. position -1 from the first nucleotide of V1 coding sequence;
- the reverse primer hybridizes to a region downstream of the sequence encoding the V3 loop of gp120 coding sequence (for instance a region of gp 120 coding sequence downstream of nucleotide 7205, i.e. position +1 from the last nucleotide of V3 coding sequence.

For instance, the forward primer may comprise or consist of the sequence E00: TAG AAA GAG CAG AAG ACA GTG GCA ATG A (SEQ ID NO:1), or E20: GGG CCA CAC ATG CCT GTG TAC CCA CAG (SEQ ID NO:6; nt 6426-6452), or a fragment thereof. The reverse primer may comprise or consist of the sequence ES8B: CAC TTC TCC AAT TGT CCC TCA (SEQ ID NO:5; nt 7658-7638), or E115 : AGA AAA ATT CCC CTC CAC AAT TAA (SEQ ID NO:7; nt 7364-7341), or a fragment thereof. The said fragments are at least 12, preferably at least 15, and more preferably at least 20 nucleotide-long.

Preferably, the step (a) of PCR amplification of the region coding for V1-V3 loops of gp120 sub-unit is performed by a RT-PCR followed by nested PCR.

According to a preferred embodiment, said PCR involves a RT-PCR of the extracted nucleic acids using primers :

E00: TAG AAA GAG CAG AAG ACA GTG GCA ATG A (SEQ ID NO:1), and
ES8B: CAC TTC TCC AAT TGT CCC TCA (SEQ ID NO:5), leading to the amplification of a 1461 bp (c)DNA spanning nt 6197-7658,

followed by a nested PCR with primers :

E20: GGG CCA CAC ATG CCT GTG TAC CCA CAG (SEQ ID NO:6), and

E115 : AGA AAA ATT CCC CTC CAC AAT TAA (SEQ ID NO:7), leading to the amplification of a 938 bp (nested c) DNA spanning nt 6426-7364 (see Figure 6).

The primers E00, ES8B, E20 and E115 have been described in the international patent application WO 02/38792.

The suitable cycling conditions RT-CPR and nested PCR may be readily determined by the one skilled in the art. The conditions cycling described in the following examples may be used, advantageously.

The vector used in step (b) is preferably "RVA-ΔV", i.e. a pNL4-3 plasmid deleted of all or part the V1-V3 region of the envelope gene, and replaced by a linker containing a suitable restriction site for linearization of the plasmid (i.e. a restriction site which will be found only once in the modified plasmid RVA-ΔV). The region to be deleted in the pNL4-3 plasmid is selected in such a way that the linearised vector contains sequences at its 5' and 3' ends which overlap the amplified nucleic acids, so that homologous recombination can occur between the vector and the amplified nucleic acids. Still preferably, said vector is the pNL4-3 plasmid deleted of nucleotides 6612-7249, and replaced by a linker containing the natural *NheI* restriction site for linearization of the plasmid. By "natural *NheI* restriction site" it is meant the *NheI* restriction site which is normally found in the V1-V3 region of pNL4-3 plasmid at positions 7250-7255.

In step (f) of the gp10-gp41ec and V1-V3 RVA assays, by "another indicator cell of the same type", is meant another cell of the indicator cell line which was used in step (e). For instance, if in step (e), a U373-CCR5 cell has been infected with the viral particles prepared from the patient's nucleic acids, then, in step (f), the U373-CCR5 cell line will be used for infection with the X4-tropic control virus. Conversely, if in step (e), a U373-CXCR4 cell has been infected with the viral particles prepared from the patient's nucleic acids, then, in step (f), the U373-CXCR4 cell line will be used for infection with the R5-tropic control virus.

As used in the instant application, a "control virus" denotes a negative control for the RVA tropism assay. For instance, a "R5-tropic control virus" denotes a R5-tropic virus which is to be assayed for infection of an indicator cell expressing CXCR4 in the presence or in the absence of a saturating dose of a CXCR4 co-receptor inhibitor. Conversely, a "X4-tropic control virus" denotes a X4-tropic virus which is to be assayed for infection of an indicator cell expressing CCR5 in the presence or in the absence of a saturating dose of a CCR5 co-receptor inhibitor. The level of marker expressed, which is associated with a control virus, represents the background level of quantification of marker expression.

Hence, theoretically if infection of the indicator cell with the viral particles prepared from the patient's nucleic acids has been 100% inhibited by the CCR5 (or CXCR4) co-receptor inhibitor, the ratio $[\text{sample/control}]_{CCR5-}$ (or $[\text{sample/control}]_{CXCR4-}$) should be equal to 1. However an interval of ratio values ranging from 0.9 to 1.1 has been retained, in order to take into account experimental variability.

In the RVA gp120-gp41ec and V1-V3 tropism assays step (f) may optionally further comprise: (f3) where said indicator cell expresses CCR5, infecting at least another indicator cell of the same type with a R5-tropic reference virus in the presence and in the absence of said saturating dose of CCR5 co-receptor inhibitor, or (f4) where said indicator cell expresses CXCR4, infecting at least another indicator cell of the same type with a X4-tropic reference virus in the presence and in the absence of said saturating dose of CXCR4 co-receptor inhibitor.

A "reference virus" denotes a positive control for the RVA tropism assay, i.e. a control which ensures that the RVA tropism assay has worked. For instance, a "R5-tropic reference virus" denotes a R5-tropic virus which is to be assayed for infection of an indicator cell expressing CCR5 in the presence or in the absence of a CCR5 co-receptor inhibitor. Conversely, a "X4-tropic reference virus" denotes a X4-tropic virus which is to be assayed for infection of an indicator cell expressing CXCR4 in the presence or in the absence of a CXCR4 co-receptor inhibitor. The use of reference viruses thus makes it possible to ensure (i) that infection of indicator cells has succeeded, hence that the RVA tropism assay should have worked if the extracted nucleic acids had been successfully amplified, and (ii) infection of the indicator cell was 100% inhibited by the saturating dose of CCR5 (or CXCR4, as appropriate) co-receptor inhibitor.

Accordingly, in the RVA gp120-gp41ec and V1-V3 tropism assays, optionally step (g) further comprises quantifying the level of marker expressed in step (f3) or (f4); and

step h) further comprises calculating the ratios

$[\text{reference/control}]_{CCR5+}$ = [level of marker expressed in step (f3) / level of marker expressed in step (f1)] in the absence of the CCR5 co-receptor inhibitor, and

$[reference/control]_{CCR5-}$ = [level of marker expressed in step (f3) / level of marker expressed in step (f1)] in the presence of the saturating dose of CCR5 co-receptor inhibitor; and/or

$[reference/control]_{CXCR4+}$ = [level of marker expressed in step (f4) / level of marker expressed in step (f2)] in the absence of the CXCR4 co-receptor inhibitor, and

$[reference /control]_{CXCR4-}$ = [level of marker expressed in step (f4) / level of marker expressed in step (f2)] in the presence of the saturating dose of CXCR4 co-receptor inhibitor,

wherein a ratio $[reference/control]_{CCR5+}$ equal or above 1.5, while the ratio $[reference/control]_{CCR5-}$ is comprised between 0.9 and 1.1 (limits included), indicates that all steps of the method have been successfully performed, and/or wherein a ratio $[reference/control]_{CXCR4+}$ equal or above 1.5, while the ratio $[reference/control]_{CXCR4-}$ is comprised between 0.9 and 1.1 (limits included), ), indicates that all steps of the method have been successfully performed.

Therefore, in a typical RVA tropism assay according to the invention, the control virus, reference virus, indicator cell, and inhibitor to be used would be as shown in Table 1.

Table 1. controls, references, cells and inhibitors used for determination of viral R5 or X4 tropism

| | R5 tropism | X4 tropism |
|---|---|---|
| control virus | X4 tropic virus (e.g. EnvX4) | R5 tropic virus (e.g. EnvR5) |
| reference virus | R5 tropic virus (e.g. EnvR5) | X4 tropic virus (e.g. EnvX4) |
| indicator cells | e.g. U373-CCR5 | e.g. U373-CXCR4 |
| coreceptor inhibitor | e.g. RANTES | e.g. AMD-3100 |

The criteria for validation of the RVA tropism assay, as well as criteria used to conclude if the virus is able to use or not (tropic or non-tropic for) the co-receptor assayed are detailed in Tables 2 and 3.

Table 2. Validation criteria for R5 tropism determination

| virus tested | CCR5 inhibitor | ratio [OD sample/OD control] | conclusion |
|---|---|---|---|
| reference | without | ≥ 1.5 | |
| | with | ~1 (0.9-1.1) | assay validated |
| control | without | ~1 (0.9-1.1) | |
| sample | without | ≥1.5 | R5 tropic R5 tropic |
| | with | ~1 (0.9-1.1) | |
| sample | without | ~1 (0.9-1.1) | not R5 tropic |
| | with | ~1 (0.9-1.1) | |

Table 3. Validation criteria for X4 tropism determination

| virus tested | CXCR4 inhibitor | ratio [OD sample/OD control] | conclusion |
|---|---|---|---|
| **reference** | without | >1.5 | |
| | with | ~1(0.9-1.1) | assay validated |

(continued)

| virus tested | CXCR4 inhibitor | ratio [OD sample/OD control] | conclusion |
|---|---|---|---|
| **control** | without | ~1 (0.9-1.1) | |
| **sample** | without | ≥1.5 | X4 tropic |
| | with | ~1 (0.9-1.1) | |
| sample | without | ~1 (0.9-1.1) | not X4 tropic |
| | with | ~1 (0.9-1.1) | |

In the above methods, the nucleic acids may be extracted from the patient's biological sample according to any method known in the art. The biological sample may be for instance blood, plasma, serum, saliva, sperm or any other secretion from a patient harbouring HIV-1 group M virus.

A "producer" cell is a cell which supports the production of viral particles but which is not permissive for HIV-1 infection. Typically, a suitable producer cell is deprived of the CD4 receptor which is necessary for the virus entry into cells. The producer cells may be for instance HeLa cells, or 293 T cells.

An "indicator" cell is a cell which produces a detectable signal upon infection with HIV-1 virus. An indicator cell may be for instance a U373-CCR5 or U373-CXCR4 cell, a P4 cell (i.e CD4-positive, HeLa cell-derived cells in which transactivation by Tat induces expression of the *Escherichia coli lacZ* gene from the HIV long terminal repeat; Charneau et al., 1994, J. Mol. Biol., 241:651-662) or a P4C5 cell (i.e P4 cells constitutively expressing the CCR5 HIV co-receptor and containing lacZ under control of the HIV-1 LTR (Amara et al., 1997, J. Exp. Med. 186:139-146). HeLa cells naturally express CXCR4.

Preferably said indicator cell is U373-CCR5 or U373-CXCR4. These cells comprise a system of expression of the gene encoding the β-galactosidase enzyme, which can only be activated by tat activation sequences expressed by the chimeric virus obtained by recombination of the vector and the amplified nucleic acids. In this case, the level of marker expressed is quantified by measuring the β-gatactosidase activity by any suitable mean, for instance using a CPRG (chlorophenolred-β-D-galactopyranoside) substrate. Briefly, when cleaved by β-galactosidase, CPRG yields chlorophenolred, which is a water-soluble red product measurable by spectrophotometry.

Examples of suitable R5-tropic (control or reference) virus that may be used in the above methods include, for instance, ADA, YU-2, JRCSF, EnvR5 (T28-R5-2) and CAR5 (T28-R5-1) viruses (the two last ones as described in Skrabal et al. (2005, J. Virol., 79(18), 11848-11857; EMBL database accession numbers AJ810480.2 and AJ810481.2).

Examples of suitable X4-tropic (control or reference) virus that may be used in the above methods include, for instance, HXB2, NL4-3, EnvX4 (T28-X4-3) and CAX4 (T28-X4-1) viruses (the two last ones as described in Skrabal et al. (2005, J. Virol., 79(18), 11848-11857; EMBL database accession numbers AJ810482.2 to AJ810483.2).

A "dual-tropic" virus, i.e. a virus having dual tropism for CCR5 and CXCR4 co-receptors may also be used as reference virus. T5-R5X4-1 ("DUAL" in the following examples) and T5-R5X4-2 (Skrabal et al., 2005, J. Virol., 79(18), 11848-11857; EMBL database accession numbers AJ810478.2) are examples of such dual tropic viruses.

Once tropism for CCR5 or CXCR4 co-receptor has be determined, the patient's viruses may be assayed for susceptibility to entry inhibitors, according to the method described below.

*Recombinant Virus Assay of susceptibility to entry inhibitors.*

A method of assaying susceptibility of HIV-1 group M viruses present in a biological sample of a patient to an entry inhibitor, may comprise the steps consisting of:

a) transfecting a producer cell with (i) nucleic acids from said biological sample of a patient which have been amplified with a pair of primers bordering a region of HIV-1 group M genome coding for all, or essentially all, the gp120 sub-unit and for the ectodomain of gp41 sub-unit, and (ii) a vector comprising the parts of an HIV-1 group M virus genome required for viral replication except for all or part of the region amplified, to obtain a chimeric virus by homologous recombination;

b) culturing said producer cell under conditions enabling the production of viral particles (during a single replication cycle);

c) incubating an indicator cell, which is liable to be infected by an HIV-1 group M virus, which comprises a marker gene which is only activated upon viral infection and which expresses CCR5, if the viruses have tropism for CCR5 co-receptor, and/or an indicator cell, which is liable to be infected by an HIV-1 group M virus, which comprises a marker gene which is only activated upon viral infection and which expresses CXCR4, if the viruses have tropism for CXCR4 co-receptor, in the absence and in the presence of an entry inhibitor;

d) infecting said indicator cells which have been incubated in the absence and in the presence of said entry inhibitor in step (c), with the viral particles obtained in step (b); and
e) quantifying the level of marker expressed by said indicator cells which have been subjected to infection in step (d);

wherein if the level of marker expressed by indicator cells which have been incubated in the presence of the entry inhibitor is decreased by comparison with the level of marker expressed by indicator cells which have been incubated in the absence of the entry inhibitor, then the viruses present in the biological sample of the patient are susceptible to said entry inhibitor.

Where the patient's viruses are dual-tropic viruses, the analysis of susceptibility to entry inhibitors is carried out on both cell types, i.e. the incubation step (step c) is performed on both an indicator cell expressing CCR5 and an indicator cell expressing CXCR4.

According to an embodiment, in the method of assaying susceptibility to an entry inhibitor, in step (c), the indicator cells are incubated in the absence and in the presence of increasing concentrations of the entry inhibitor. Step (e) of the method then further comprises determination of an inhibitory concentration index from the quantified levels of marker expressed by the indicator cells.

Thus, the method of assaying susceptibility may be a method of determining the inhibitory concentration index of an entry inhibitor towards the HIV-1 group M viruses present in a biological sample of a patient, which method comprises:

a) transfecting a producer cell with (i) nucleic acids from said biological sample of a patient which have been amplified with a pair of primers bordering a region of HIV-1 group M genome coding for all, or essentially all, the gp120 sub-unit and for the ectodomain of gp41 sub-unit, and (ii) a vector comprising the parts of an HIV-1 group M virus genome required for viral replication except for all or part of the region amplified, to obtain a chimeric virus by homologous recombination;
b) culturing said producer cell under conditions enabling the production of viral particles (during a single replication cycle);
c) incubating an indicator cell, which is liable to be infected by an HIV-1 group M virus, which comprises a marker gene which is only activated upon viral infection and which expresses CCR5, if the viruses have tropism for CCR5 co-receptor, and/or an indicator cell, which is liable to be infected by an HIV-1 group M virus, which comprises a marker gene which is only activated upon viral infection and which expresses CXCR4, if the viruses have tropism for CXCR4 co-receptor, in the absence and in the presence of increasing concentrations of an entry inhibitor;
d) infecting said indicator cells, which have been incubated in the absence and in the presence of said increasing concentrations of entry inhibitor in step (c), with the viral particles obtained in step (b); and
e) quantifying the level of marker expressed by said indicator cells which have been subjected to infection in step (d), and determining the inhibitory concentration index of said entry inhibitor.

Where the patient's viruses are dual-tropic viruses, the method of determining inhibitory concentration index is carried out on both cell types, i.e. the incubation step (step c) is performed on both an indicator cell expressing CCR5 and an indicator cell expressing CXCR4.

The determination of an inhibitory index is within the ordinary skills of the one skilled in the art. For instance, the concentration of a drug that inhibits viral replication by 50% (inhibitory concentration 50, IC50) or 90% (IC90) is calculated by determining the percentage of infection in presence of inhibitor (at different concentrations), as compared to 100% of infection (viral infection in absence of inhibitor), which allows the design of a sigmoidal inhibition curve (% of infection vs concentration of inhibitor). Inhibitory concentration indexes may be deduced from this curve very simply by determining the x-coordinate (inhibitor concentration) of the point on the curve having y-coordinate equal to 50% (IC50) or 90% (IC90).

Accordingly the invention further provides a method of assaying tropism of HIV-1 group M viruses present in a biological sample of a patient for CCR5 and/or CXCR4 co-receptor and susceptibility of said viruses to an entry inhibitor, which method comprises the steps consisting of:

a) subjecting nucleic acids extracted from said biological sample of a patient to a PCR amplification with a pair of primers bordering a region of HIV-1 group M genome coding for all, or essentially all, the gp120 sub-unit and for the ectodomain of gp41 sub-unit;
b) preparing a vector comprising the parts of an HIV-1 group M virus genome required for viral replication except for all or part of the region amplified in step (a);
c) transfecting a producer cell with the amplified nucleic acids of step (a) and the vector prepared in step (b) to obtain a chimeric virus by homologous recombination;
d) culturing said producer cell under conditions enabling the production of viral particles (during a single replication cycle);
e) infecting an indicator cell, which is liable to be infected by an HIV-1 group M virus, which comprises a marker gene which is only activated upon viral infection and which expresses either CCR5 or CXCR4 co-receptor, with the viral particles obtained in step (d),
wherein (e1) said infection is carried out in the presence and in the absence of a saturating dose of a CCR5 co-receptor inhibitor where said indicator cell expresses CCR5, or (e2) said infection is carried out in the presence and in the absence of a saturating dose of a CXCR4 co-receptor inhibitor where said indicator cell expresses CXCR4;
f) separately, (f1) where said indicator cell expresses CCR5, infecting at least another indicator cell of the same type with a X4-tropic control virus in the presence and in the absence of said saturating dose of CCR5 co-receptor inhibitor,
or (f2) where said indicator cell expresses CXCR4, infecting at least another indicator cell of the same type with a R5-tropic control virus in the presence and in the absence of said saturating dose of CXCR4 co-receptor inhibitor;
g) quantifying the level of marker expressed in steps (e1) and (f1), and/or in steps (e2) and (f2); and
h) calculating the ratios

$[sample/control]_{CCR5+}$ = [level of marker expressed in step (e1) / level of marker expressed in step (f1)] in the absence of the CCR5 co-receptor inhibitor, and

$[sample/control]_{CCR5-}$ = [level of marker expressed in step (e1) / level of marker expressed in step (f1)] in the presence of said saturating dose of CCR5 co-receptor inhibitor; and/or

$[sample/control]_{CXCR4+}$ = [level of marker expressed in step (e2) / level of marker expressed in step (f2)] in the absence of the CXCR4 co-receptor inhibitor, and

$[sample/control]_{CXCR4-}$ = [level of marker expressed in step (e2) / level of marker expressed in step (f2)] in the presence of said saturating dose of CXCR4 co-receptor inhibitor;

wherein a ratio $[sample/control]_{CCR5+}$ equal or above 1.5, while the ratio $[sample/control]_{CCR5-}$ is comprised between 0.9 and 1.1 (limits included), is indicative of the presence of HIV-1 group M viruses, in the biological sample from the patient, having tropism for CCR5 co-receptor, and/or
wherein a ratio $[sample/control]_{CXCR4+}$ equal or above 1.5, while the ratio $[sample/control]_{CXCR4-}$ is comprised between 0.9 and 1.1 (limits included), is indicative of the presence of HIV-1 group M viruses, in the biological sample from the patient, having tropism for CXCR4 co-receptor; and
i) transfecting a producer cell with the amplified nucleic acids of step (a) and the vector prepared in step (b) to obtain a chimeric virus by homologous recombination;
j) culturing said producer cell of step (j) under conditions enabling the production of viral particles during a single replication cycle;
k) incubating an indicator cell, which is liable to be infected by an HIV-1 group M virus, which comprises a marker gene which is only activated upon viral infection and which expresses CCR5, if the viruses have tropism for CCR5 co-receptor, and/or an indicator cell, which is liable to be infected by an HIV-1 group M virus, which comprises a marker gene which is only activated upon viral infection and which expresses CXCR4, if the viruses have tropism for CXCR4 co-receptor, in the absence and in the presence of an entry inhibitor;
l) infecting said indicator cells which have been incubated in the absence and in the presence of said entry inhibitor in step (k), with the viral particles obtained in step (j); and
m) quantifying the level of marker expressed by said indicator cells which have been subjected to infection in step (l);

wherein if the level of marker expressed by indicator cells which have been incubated in the presence of the entry inhibitor is decreased by comparison with the level of marker expressed by indicator cells which have been incubated in the absence of the entry inhibitor, then the viruses present in the biological sample of the patient are susceptible to said entry inhibitor.

Where the viruses have been identified as dual-tropic viruses, the analysis of susceptibility to entry inhibitors is carried out on both cell types, i.e. the incubation step (step k) is performed on both an indicator cell expressing CCR5 and an indicator cell expressing CXCR4.

In the method of assaying tropism for CCR5 and/or CXCR4 co-receptor and susceptibility to an entry inhibitor, step (k) may be performed by incubating the indicator cells in the absence and in the presence of increasing concentrations of the entry inhibitor, and step (m) may further comprise determining an inhibitory concentration index of said entry inhibitor.

Thus, the method may be a method of assaying tropism of HIV-1 group M viruses present in a biological sample of a patient for CCR5 and/or CXCR4 co-receptor and of determining inhibitory concentration index of said HIV-1 group M viruses present in the biological sample of a patient towards an entry inhibitor, which method comprises the steps consisting of:

a) subjecting nucleic acids extracted from said biological sample of a patient to a PCR amplification with a pair of primers bordering a region of HIV-1 group M genome coding for all, or essentially all, the gp120 sub-unit and for the ectodomain of gp41 sub-unit;
b) preparing a vector comprising the parts of an HIV-1 group M virus genome required for viral replication except for all or part of the region amplified in step (a);
c) transfecting a producer cell with the amplified nucleic acids of step (a) and the vector prepared in step (b) to obtain a chimeric virus by homologous recombination;
d) culturing said producer cell under conditions enabling the production of viral particles (during a single replication cycle?);
e) infecting an indicator cell, which is liable to be infected by an HIV-1 group M virus, which comprises a marker gene which is only activated upon viral infection and which expresses either CCR5 or CXCR4 co-receptor, with the viral particles obtained in step (d),
wherein (e1) said infection is carried out in the presence and in the absence of a saturating dose of a CCR5 co-receptor inhibitor where said indicator cell expresses CCR5, or (e2) said infection is carried out in the presence and in the absence of a saturating dose of a CXCR4 co-receptor inhibitor where said indicator cell expresses CXCR4;
f) separately, (f1) where said indicator cell expresses CCR5, infecting at least another indicator cell of the same type with a X4-tropic control virus in the presence and in the absence of said saturating dose of CCR5 co-receptor inhibitor,
or (f2) where said indicator cell expresses CXCR4, infecting at least another indicator cell of the same type with a R5-tropic control virus in the presence and in the absence of said saturating dose of CXCR4 co-receptor inhibitor;
g) quantifying the level of marker expressed in steps (e1) and (f1), and/or in steps (e2) and (f2); and
h) calculating the ratios

$[\text{sample/control}]_{CCR5+}$ = [level of marker expressed in step (e1) / level of marker expressed in step (f1)] in the absence of the CCR5 co-receptor inhibitor, and

$[\text{sample/control}]_{CCR5-}$ = [level of marker expressed in step (e1) / level of marker expressed in step (f1)] in the presence of said saturating dose of CCR5 co-receptor inhibitor; and/or

$[\text{sample/control}]_{CXCR4+}$ = [level of marker expressed in step (e2) / level of marker expressed in step (f2)] in the absence of the CXCR4 co-receptor inhibitor, and

$[sample/control]_{CXCR4-}$ = [level of marker expressed in step (e2) / level of marker expressed in step (f2)] in the presence of said saturating dose of CXCR4 co-receptor inhibitor;

wherein a ratio $[sample/control]_{CCR5+}$ equal or above 1.5, while the ratio $[sample/control]_{CCR5-}$ is comprised between 0.9 and 1.1 (limits included), is indicative of the presence of HIV-1 group M viruses, in the biological sample from the patient, having tropism for CCR5 co-receptor, and/or
wherein a ratio $[sample/control]_{CXCR4+}$ equal or above 1.5, while the ratio $[sample/control]_{CXCR4-}$ is comprised between 0.9 and 1.1 (limits included), is indicative of the presence of HIV-1 group M viruses, in the biological sample from the patient, having tropism for CXCR4 co-receptor; and
i) transfecting a producer cell with the amplified nucleic acids of step (a) and the vector prepared in step (b) to obtain a chimeric virus by homologous recombination;
j) culturing said producer cell of step (j) under conditions enabling the production of viral particles during a single replication cycle;
k) incubating an indicator cell, which is liable to be infected by an HIV-1 group M virus, which comprises a marker gene which is only activated upon viral infection and which expresses CCR5, if the viruses have tropism for CCR5 co-receptor, and/or an indicator cell, which is liable to be infected by an HIV-1 group M virus, which comprises a marker gene which is only activated upon viral infection and which expresses CXCR4, if the viruses have tropism for CXCR4 co-receptor, in the absence and in the presence of increasing concentrations of an entry inhibitor;
l) infecting said indicator cells, which have been incubated in the absence and in the presence of said increasing concentrations of entry inhibitor in step (k), with the viral particles obtained in step (i); and
m) quantifying the level of marker expressed by said indicator cells which have been subjected to infection in step (l), and determining the inhibitory concentration index of said entry inhibitor.

Where the patient's viruses are dual-tropic viruses, the method of determining inhibitory concentration index is carried out on both cell types, i.e. the incubation step (step c) is performed on both an indicator cell expressing CCR5 and an indicator cell expressing CXCR4.
In the above methods of assaying tropism for CCR5 and/or CXCR4 co-receptor and susceptibility to an entry inhibitor (in particular of determining inhibitory concentration index), advantageously:

- the producer cells of steps (c) and (i) are cells from a same cell line; and/or
- the indicator cells of steps (e) and (k) are cells from a same cell line.

All definitions, embodiments, etc, described in the Section "Recombinant Virus Assay of R5 and/or X4 tropism" also apply to the instant methods of assaying tropism for CCR5 and/or CXCR4 co-receptor and susceptibility to an entry inhibitor (in particular of determining inhibitory concentration index).
The entry inhibitor to be assayed may be an inhibitor interacting between gp120 and CD4, or a co-receptor inhibitor, or a fusion inhibitor.
The range of concentrations of inhibitor which may be used in the assay can be readily determined by the one skilled in the art. The one skilled in the art would typically determine first the concentration at which toxicity starts to be observed on the indicator cells of step (k). The inhibitor would then be assayed with non-toxic concentrations.
The methods of assaying tropism for CCR5, and/or CXCR4, co-receptor and/or susceptibility to an entry inhibitor according to the invention should help the clinician to define an appropriate therapeutic treatment (in particular a treatment including a co-receptor inhibitor), and/or to determine whether such a treatment is appropriate for the patient. Indeed, if the viruses of the patient show a dual tropism pattern (R5/X4), administration of a CCR5 inhibitor might induce mutations towards a marked X4-tropic pattern with a risk of disease progression.
The methods of assaying tropism for CCR5 and/or CXCR4, co-receptor also make it possible to monitor efficacy of a treatment. Actually, analysis of the pattern of R5-tropic and X4-tropic viruses in the course of a therapeutic treatment is valuable for the clinician who can ensure him(her)self that a switch from R5-tropic to X4-tropic viruses does not occur.

*Kits*

The invention further provides kits for implementing the above described methods.
In particular, a kit for assaying susceptibility of HIV-1 group M viruses present in a biological sample of a patient to CCR5 and/or CXCR4 co-receptor inhibitors, and/or of assaying tropism of said HIV-1 group M viruses for CCR5 and/or CXCR4 co-receptor, may comprise:

a) at least one pair of primers bordering a region of HIV-1 group M genome coding for all, or essentially all, the gp120 sub-unit and for the ectodomain of gp41 sub-unit;
b) a vector comprising the parts of an HIV-1 group M virus genome required for viral replication except for all or part of the region amplified with the pair of primers defined in (a);
c) a producer cell which is suitable to support the production of HIV-1 viral particles, and which is preferably not permissive for HIV-1 infection;
d) an indicator cell which is liable to be infected by an HIV-1 group M virus, which comprises a marker gene which is only activated upon viral infection and which expresses either CCR5 or CXCR4 co-receptor;
e) a R5-tropic virus and a CCR5 co-receptor inhibitor, and/or a X4-tropic virus and a CXCR4 co-receptor inhibitor;
f) optionally, reagents required to carry out PCR amplification; and
g) optionally, reagents required to quantify the level of marker expressed.

Said kit may comprise the following primer pairs:

- a pair of primers having sequences SEQ ID NO:1 and SEQ ID NO:2, and
- a pair of primers having sequences SEQ ID NO:3 and SEQ ID NO:4.

Said kit may also comprise a pNL4-3 plasmid deleted of nucleotides 6480-8263 and replaced by a linker containing the *MluI* restriction site.
It is further provided a kit for assaying tropism of HIV-1 group M viruses present in a biological sample of a patient for CCR5 and/or CXCR4 co-receptor, which comprises:

a) a least one pair of primers bordering the entire region of HIV-1 group M genome coding for V1-V3 loops of gp120 sub-unit;
b) a vector comprising the parts of an HIV-1 group M virus genome required for viral replication except for all or part of the region amplified with the pair of primers defined in (a);
c) a producer cell which is suitable to support the production of HIV-1 viral particles, and which is preferably not permissive for HIV-1 infection;
d) an indicator cell which is liable to be infected by an HIV-1 group M virus, which comprises a marker gene which is only activated upon viral infection and which expresses either CCR5 or CXCR4 co-receptor;
e) a R5-tropic virus and a CCR5 co-receptor inhibitor, and/or a X4-tropic virus and a CXCR4 co-receptor inhibitor;
f) optionally, reagents required to carry out PCR amplification; and
g) optionally, reagents required to quantify the level of marker expressed.
Said kit may comprise the following primer pairs:

- a pair of primers having sequences SEQ ID NO:1 and SEQ ID NO:5, and
- a pair of primers having sequences SEQ ID NO:6 and SEQ ID NO:7.

Said kit may also comprise a pNL4-3 plasmid deleted of nucleotides 6612-7249, containing the natural *NheI* restriction site for linearization of the plasmid.
In the kits of the invention, a reagent required to quantify the level of marker expressed may be for instance the CPRG substrate.
The R5-tropic control/reference virus may be, for instance, EnvR5, CAR5, ADA, YU-2, or JR-CSF..
The X4-tropic reference virus may be, for instance, EnvX4, CAX4, HXB2.
A CCR5 co-receptor inhibitor may be, for instance, RANTES, or antibodies. 3A9, SD7, 45502.111, 45549.111, 45531.111 and 45523.111 (http://www.aidsreagent.org)
A CXCR4 co-receptor inhibitor may be, for instance, AMD3100, antibodies or natural ligands of CXCR4.
The invention will be further illustrated in view of the following figures and

examples.

## FIGURES

Figures 1-3 display the genomic sequence of HIV-1 strain NL43 from nucleotide 6171 to nucleotide 9090.
Figure 4 is a diagrammatic representation of the principle of the RVA assay.
Figure 5 shows the results of RVA assays (gp120-gp41ec) performed on a mixture of viral envelopes from reference viruses NL4-3 and JR-CSF, with increasing percentages of X4 tropic virus (NL43).

Figure 6 shows the results of RVA assays (gp120-gp41ec) performed on a mixture of viral envelopes from primary viruses CAX4 and EnvR5, with increasing percentages of the X4 tropic virus (CAX4).

Figure 7 shows the results of RVA assays (V1-V3) performed on a mixture of primary envelopes CAX4 and EnvR5, and with increasing percentages of X4 tropic virus (CAX4).

## EXAMPLES

### Example 1: amplification of gp120/gp41ec by RT-PCR and nested PCR

The SuperScriptTM III One-Step RT-PCR System with Platinum® Taq DNA Polymerase (Invitrogen Life Technologies Cat. No. 12574-018 or 12574-026) has been used for performing RT-PCR.

A RT-PCR mix is prepared as follows:

| | |
|---|---|
| 2X Reaction mix | 25 µl |
| primer E00 (10 µM) | 1 µl |
| primer E01 (10 µM) | 1 µl |
| SuperscriptTM III RT/Platinum® Taq Mix | 2 µl |
| water | ad 45 µl |

where the primers E00 and E01 have the following sequences:

E00: TAG AAA GAG CAG AAG ACA GTG GCA ATG A (SEQ ID NO:1), and
E01-: TCC AGT CCC CCC TTT TCT TTT AAA AA (SEQ ID NO:2).

45 µl of this mix are put in each PCR tube, then 5 µl of viral RNA are added, with pipette tips and the pipette reserved for RNA. Viral RNA has been prepared with QIAamp Viral RNA Mini Kit (Qiagen 52904 (x50) or 52906 (x250)) according to the manufacturer's instructions.

EnvR5, EnvX4, CAR5, CAX4 and DUAL are used as reference and control viruses to amplify.

The RT-PCR tubes are placed in the thermocycler Hybaid PCR Express or the icycler Biorad, and amplification is carried out as follows:

| | | |
|---|---|---|
| 1 cycle: | 45°C | 30 min |
| | 94°C | 2 min |
| 40 cycles: | 94°C | 15 sec |
| | 55°C | 30 sec |
| | 68°C | 3 min |
| 1 cycle: | 68°C | 10 min |
| | 4°C | |

For nested PCR, Accuprime™ Pfx SuperMix (Invitrogen Life Technologies Cat. No. 12344-040) was used.

A mix for the nested PCR is prepared as follows:

| | |
|---|---|
| AccuPrimeTM Pfx SuperMix | 22.5 µl |
| Primer E10 (10µM) | 0.5 µl |
| Primer FuB (10µM) | 0.5 µl |

where the primers E10 and FuB have the following sequences:

E10: TTG TGG GTC ACA GTC TAT TAT GGG GT (SEQ ID NO:3), and
FuB: GGT GGT AGC TGA AGA GGC ACA GG (SEQ ID NO:4).

23.5 µl of this mix are placed in PCR tubes. Under a molecular biology hood, 3 µl of the RT-PCR reaction mixture are added in the PCR tubes. The nested PCR is carried out by placing the tubes in the thermocycler Hybaid PCR Express or the icycler Biorad, with the following program:

| | |
|---|---|
| 1 cycle: | 95°C 5 min |
| 35 cycles: | 95°C 15 sec |
| | 55°C 30 sec |
| | 68°C 2 min 30 sec |
| 1 cycle: | 68°C 10 min |
| | 15°C |

**Example 2: amplification of V1V3 by RT-PCR and nested PCR**

A RT-PCR mix is prepared as follows

| | |
|---|---|
| 2X Reaction mix | 25 μl |
| primer E00 (10 μM) | 1 μl |
| primer ES8B (10 μM) | 1 μl |
| SuperscriptTM III RT/Platinum® Taq Mix | 2 μl |
| water | ad 45 μl |

where the primers E00 and ES8B have the following sequences:

E00: TAG AAA GAG CAG AAG ACA GTG GCA ATG A (SEQ ID NO:1), and
ES8B: CAC TTC TCC AAT TGT CCC TCA (SEQ ID NO:5).

45 μl of this mix are put in each PCR tube, then 5 μl of viral RNA, with pipette tips and the pipette reserved for RNA.

EnvR5, EnvX4, CAR5, CAX4 and DUAL are used as reference and control viruses to amplify.

The RT-PCR tubes are placed in the thermocycler Hybaid PCR Express or the icycler Biorad, and amplification is carried out as follows:

| | |
|---|---|
| 1 cycle: | 45°C 30 min |
| | 94°C 2 min |
| 40 cycles: | 94°C 15 sec |
| | 52°C 30 sec |
| | 68°C 1 min 45 sec |
| 1 cycle: | 68°C 10 min |
| | 4°C |

A mix for the nested PCR is prepared as follows:

| | |
|---|---|
| AccuPrimeTM Pfx SuperMix | 22.5 μl |
| Primer E20 (10μM) | 0.5 μl |
| Primer E115 (10μM) | 0.5 μl |

where the primer E20 and E115 have the following sequences: E20: GGG CCA CAC ATG CCT GTG TAC CCA CAG (SEQ ID NO:6), and E115 : AGA AAA ATT CCC CTC CAC AAT TAA (SEQ ID NO:7).

23.5 μl of this mix are placed in PCR tubes. Under a molecular biology hood, 3 μl of the RT-PCR reaction mixture are added in the PCR tubes. The nested PCR is carried out by placing the tubes in the thermocycler Hybaid PCR Express or the icycler Biorad, with the following program:

| | |
|---|---|
| 1 cycle: | 95°C 5 min |
| 35 cycles: | 95°C 15 sec |
| | 54°C 30 sec |
| | 68°C 1 min |
| 1 cycle: | 68°C 10 min |

(continued)

15°C

**<u>Example 3:</u> verification of PCR products**

The PCR products are controlled on an agarose gel at 1 %. The expected size of the PCR product is 2202 nt for gp120/gp41ec, and 938 nt for V1V3.

The RT-PCR tubes of positive samples are stored in a clean room at 4°C. The RT-PCR tubes for samples giving negative, weak or non-specific products are discarded, unless they represent the best result obtained for the sample.

The nested PCR tubes for positive samples are stored at 4°C, until purification. The nested PCR tubes for samples giving negative, weak or non-specific products are also kept until the result of the next PCR is available.

**<u>Example 4:</u> transfection of producer cells (293T cells)**

One T25 flask of 293T cells is prepared for each sample to be tested and for the 2 references and the 3 controls. Cells are added in sufficient number to give a confluence of 70 to 80% on the transfection day. The complete medium for 293T cells is 500 ml DMEM (Dulbecco's Modified Eagles Medium with Glutamax-I), with 50 ml Foetal calf serum (FCS), 2.5 ml P/S (Penicillin (10000 IU/ml) and Streptomycin (10000 μg/ml)),. The flasks are incubated at 37°C, 5% CO2.

On the day of transfection, the condition of cells in the T25 flasks is verified and the medium changed at least 3 to 4 hours before transfection (4 ml/ T25).

The plasmid used for transfection is either RVA-Δenv, pNL4-3 deleted of the gp120-gp41ec region of the envelope gene (nt 6480-8263) and replaced by a linker containing the *MluI* restriction site for linearization of the plasmid, or RVA-ΔV, pNL4-3Δ deleted of the V1-V3 region of the envelope gene (nt 6612-7249), containing the natural *NheI* restriction site for linearization of the plasmid.

In a labelled sterile microtube, a transfection mix is prepared for each sample using the volumes given in the table below:

| Reagents | Volume / sample |
|---|---|
| vector linearized 1μg/μl | 8 μl |
| 1M $CaCl_2$ | 125 μl |
| Tris-EDTA pH 8.0 (1/10) | 352 μl |
| Total | 485 μl |

Then 15 μl of the appropriate PCR product (approximately 0.8 μg of DNA) are added.

For each sample, 500 μl of HBS 2X (280 mM NaCl, 10 mM KCl, 1,5 mM $Na_2HPO4$, 50 mM Hepes) are placed into a second labelled sterile microtube.

The mixture of the transfection mix and PCR product is added, drop by drop, into the tube containing the HBS 2X, while lightly vortexing. This transfection precipitate is added to the flasks of 293T cells. The presence of a precipitate on the cells is verified and the flasks are incubated 16-18 hours at 37°C, 5% CO2.

All the medium is aspirated using a pipette and the cells are washed once with sterile PBS 1X. 4 ml of fresh medium per sample are added and the flasks are incubated 24 hours at 37°C, 5% CO2.

**<u>Example 4:</u> infection of indicator cells**

The condition, confluence and mortality of the transfected 293T cells are verified. The entire medium is removed from each flask and transferred into a 15 ml conical tube to be clarified. The tubes are centrifuged at 1800 rpm for 10 to 15 minutes. The supernatants are kept at +4°C until the test is completed.

The indicator cells U373-CCR5 and U373-CXCR4 have been prepared by passing in T75 flasks (1 T75 flask for 8 plates to be prepared on the transfection day) and incubating the flasks at 37°C, 5% CO2. The complete medium for U373-CCR5 and U373-CXCR4 cells is 500 ml DMEM with 50 ml FCS, 2.5 ml P/S, 0.5 ml puromycine (10 mg/ml), 1 ml hygromycine (50 mg/ml),

On the day of infection, the condition of the indicator U373-CCR5 and U373-CXCR4 cells is verified. CXCR4- and CCR5-inhibitors are prepared at 4x their final concentrations used in the test (e.g. 4x AMD-3100 at 8 μg/ml).

The medium of plates of U373-CCR5 and U373-CXCR4 cells is aspirated and 50 μl of medium containing DEAE dextran (4X, for a final concentration of 2 μg/ml per well/ 200 μl) are distributed simultaneously to avoid drying of the cells, and 50 μl of medium or of the co-receptor inhibitor on half of the plate.

50 and 100 μl of virus are transferred and medium is added up to 200 μl in each well of the U373-CCR5 and U373-CXCR4 plates. Each plate includes one R5-tropic control/reference virus (EnvR5 or CAR5), as well as one X4-tropic control/reference virus (EnvX4 or CAX4) which will allow the analysis of data : The presence of all references (EnvR5, EnvX4, CAR5, CAX4 and DUAL) on at least one plate per cell-line and per batch allows the general validation of the experiment. The reference serves thus as positive control for the validation of the test (e.g., EnvR5 on U373-CCR5 or EnvX4 on U373-CXCR4). The controls (EnvR5 on U373-CXCR4 or EnvX4 on U373-CCR5) serve for the calculation of the ratio [OD sample/OD control].

Infection is carried out using two different doses of viral input (50 and 100 μl) in triplicates, with replica plates in presence of co-receptor inhibitors (RANTES (Sigma Aldrich R6267) or AMD-3100 (Sigma Aldrich A5602)).

The plates are incubated for 40 h at 37°C, 5% CO2.

**<u>Example 6:</u> CPRG test**

The β-galactosidase produced by the infected U373-CCR5 or U373-CXCR4 cells is quantified: the level of marker expressed is quantified by measuring the β-galactosidase activity by using a CPRG (chlorophenolred-β-D-galactopyranoside) substrate. Briefly, when cleaved by β-galactosidase, CPRG yields chlorophenolred, which is a water-soluble red product measurable by spectrophotometry. Briefly, cells are lysed and the substrate (CPRG diluted in lysis buffer) is added before OD measurement.

A sample is considered positive, when the ratio [OD sample/OD control] reaches or exceeds 1.5, and when specific inhibition is observed in presence of the corresponding co-receptor inhibitor at saturating dose (e.g. RANTES, AMD-3100).

**<u>Example 7:</u> RVA tropism assay with the cassette Env (gp120-gp41ec)**

The RVA tropism test has been realized on PCR products obtained from mixtures of proviral plasmids of different tropism (characterized reference viruses NL4-3/JR-CSF and mixtures of plasmids carrying cloned envelopes from patients' plasma samples: CAX4/EnvR5 at different percentages (0%, 5%, 10%, 15%, 20%, 30%, 40%, 50%, 100% of X4 tropic virus). The PCR products of independent PCR reactions have been tested in independent tropism tests.

Results representative of 3 independent recombinant virus assays, realized with PCR products obtained in independent PCR reactions, are shown on Figures 5 and 6. The gp120-gp41ec fragment was amplified on mixtures of plasmids NL43 and JR-CSF for reference, and CAX4/EnvR5 mixtures for primary viruses.

When mixing viral envelopes from reference viruses NL4-3 and JR-CSF, 5% of X4 tropic virus were detectable, and the infection was inhibited (100% of inhibition) using AMD3100 (Figure 5). This inhibition proves the specificity of the CPRG signal and hence of the infection via CXCR4.

In a mixture of primary viruses of comparable infectivity (i.e. production of infectious viral particles induces comparable CPRG signals on U373-CCR5 and U373-CXCR4 cells), X4 tropic viruses are specifically detectable at a percentage of 5% (3 experiments) (Figure 6).

**<u>Example 8:</u> RVA tropism assay with the cassette V1V3**

The inventors presumed that sensitivity of the test for non-B subtypes might increase when using a smaller fragment of the envelope, the V1V3 or tropism-determinant region: due to amplification of the smaller fragment, not only PCR, but also recombination, viral production and hence infection of indicator cells might be more sensitive. PCR products spanning the V1V3 region (~900 nt) were obtained on plasmid mixtures of reference and primary envelopes.

Using this V1V3 cassette, detection of primary X4 tropic virus in a mixture of viruses with comparable infectivity was 5% (Figure 7).

**<u>Example 9:</u> PCR and RVA success rate for group M non-B samples**

The test of the invention allows the amplification of subtype B as well as non-B samples, with high specificity and sensitivity.

The PCR success rate for most prevalent European non-B subtypes with the 2 different cassettes was comparable, ranging from 57 to 100% for gp120-gp41ec, and from 67 to 96% for the V1V3 cassette.

The overall RVA success rate for tropism determination using one or the other cassette ranged from 42 to 98%, depending on the subtype.

Table 4: RVA tropism assay rate of success

| Subtype | Prevalence in Europe | Number of samples available | Tested in PCR gp120-gp41ec | PCR Success rate | Tested in PCR V1V3 | PCR Success rate | Tested in RVA | RVA Success rate | % R5 | %R5X4 | %X4 |
|---|---|---|---|---|---|---|---|---|---|---|---|
| C | 37,5% | 143 | 55 | 95% | 95 | 75% | 81 | 98% | 90% | 7.5% | 2.5% |
| G | 15,5% | 59 | 38 | 87% | 32 | 81% | 40 | 70% | 93% | 7% | |
| CRF02_AG | 12,5% | 61 | 30 | 87% | 45 | 96% | 46 | 87% | 90% | 7.5% | 2.5% |
| A | 11,0% | 39 | 21 | 57% | 24 | 67% | 19 | 42% | 86% | 14% | |
| CRF01_AE | 9,0% | 59 | 19 | 89% | 32 | 88% | 41 | 56% | 100% | | |
| D | 3,5% | 21 | 12 | 83% | 14 | 71% | 15 | 73% | 75% | 12.5% | 12.5% |
| F | 3,5% | 26 | 8 | 63% | 20 | 70% | 16 | 75% | 82% | 18% | |
| J | 2,5% | 9 | 4 | 100% | 6 | 83% | 6 | 67% | 75% | | 25% |

## Claims

1. A method of assaying tropism of HIV-1 group M viruses present in a biological sample of a patient for CCR5 and/or CXCR4 co-receptor, which method comprises the steps consisting of:

   a) subjecting nucleic acids extracted from said biological sample of a patient to a PCR amplification with a pair of primers bordering a region of HIV-1 group M genome coding for all, or essentially all, the gp120 sub-unit and for the ectodomain of gp41 sub-unit;
   b) preparing a vector comprising the parts of an HIV-1 group M virus genome required for viral replication except for all or part of the region amplified in step (a);
   c) transfecting a producer cell with the amplified nucleic acids of step (a) and the vector prepared in step (b) to obtain a chimeric virus by homologous recombination;
   d) culturing said producer cell under conditions enabling the production of viral particles;
   e) infecting an indicator cell, which is liable to be infected by an HIV-1 group M virus, which comprises a marker gene which is only activated upon viral infection and which expresses either CCR5 or CXCR4 co-receptor, with the viral particles obtained in step (d),
   wherein (e1) said infection is carried out in the presence and in the absence of a saturating dose of a CCR5 co-receptor inhibitor where said indicator cell expresses CCR5, or (e2) said infection is carried out in the presence and in the absence of a saturating dose of a CXCR4 co-receptor inhibitor where said indicator cell expresses CXCR4;
   f) separately, (f1) where said indicator cell expresses CCR5, infecting at least another indicator cell of the same type with a X4-tropic control virus in the presence and in the absence of said saturating dose of CCR5 co-receptor inhibitor, or (f2) where said indicator cell expresses CXCR4, infecting at least another indicator cell of the same type with a R5-tropic control virus in the presence and in the absence of said saturating dose of CXCR4 co-receptor inhibitor;
g) quantifying the level of marker expressed in steps (e1) and (f1), and/or in steps (e2) and (f2); and
h) calculating the ratios

$[sample/control]_{CCR5+}$ = [level of marker expressed in step (e1) / level of marker expressed in step (f1)] in the absence of the CCR5 co-receptor inhibitor, and

$[sample/control]_{CCR5-}$ = [level of marker expressed in step (e1) / level of marker expressed in step (f1)] in the presence of said saturating dose of CCR5 co-receptor inhibitor; and/or

$[sample/control]_{CXCR4+}$ = [level of marker expressed in step (e2) / level of marker expressed in step (f2)] in the absence of the CXCR4 co-receptor inhibitor,

and

$[sample/control]_{CXCR4-}$ = [level of marker expressed in step (e2) / level of marker expressed in step (f2)] in the presence of said saturating dose of CXCR4 co-receptor inhibitor;

wherein a ratio $[sample/control]_{CCR5+}$ equal or above 1.5, while the ratio $[sample/control]_{CCR5-}$ is comprised between 0.9 and 1.1 (limits included), is indicative of the presence of HIV-1 group M viruses, in the biological sample from the patient, having tropism for CCR5 co-receptor, and/or
wherein a ratio $[sample/control]_{CXCR4+}$ equal or above 1.5, while the ratio $[sample/control]_{CXCR4-}$ is comprised between 0.9 and 1.1 (limits included), is indicative of the presence of HIV-1 group M viruses, in the biological sample from the patient, having tropism for CXCR4 co-receptor.

**2.** The method according to claim 1, wherein said pair of primers consists of:

- a forward primer comprising or consisting of the sequence SEQ ID NO:1 or SEQ ID NO:3, or a fragment thereof; and
- a reverse primer comprising or consisting of the sequence SEQ ID NO:2, or SEQ ID NO:4, or a fragment thereof.

**3.** The method according to claim 1 or 2, wherein said PCR amplification comprises a RT-PCR of said extracted nucleic acids with a pair of primers having sequences SEQ ID NO:1 and SEQ ID NO:2, followed by a nested PCR with a pair of primers having sequences SEQ ID NO:3 and SEQ ID NO:4.

**4.** The method according to any of claims 1 to 3, wherein the vector used in step (b) is a pNL4-3 plasmid deleted of all or part the gp120-gp41ec region of the envelope gene which has been replaced by a linker containing a suitable restriction site for linearization of the plasmid.

**5.** The method according to claim 4, wherein said vector is the pNL4-3 plasmid deleted of nucleotides 6480-8263 and replaced by a linker containing the *MluI* restriction site.

**6.** A method of assaying tropism of HIV-1 group M viruses present in a biological sample of a patient for CCR5 and/or CXCR4 co-receptor, which method comprises the steps consisting of:

a) subjecting nucleic acids extracted from said biological sample of a patient to a PCR amplification with a pair of primers bordering the entire region of HIV-1 group M genome coding for V1-V3 loops of gp120 sub-unit;
b) preparing a vector comprising the parts of an HIV-1 group M virus genome required for viral replication except for the region coding for V1-V3 loops of gp120 sub-unit;
c) transfecting a producer cell with the amplified nucleic acids of step (a) and the vector prepared in step (b) to obtain a chimeric virus by homologous recombination;
d) culturing said producer cell under conditions enabling the production of viral particles;
e) infecting an indicator cell, which is liable to be infected by an HIV-1 group M virus, which comprises a marker gene which is only activated upon viral infection and which expresses either CCR5 or CXCR4 co-receptor, with the viral particles obtained in step (d),
wherein (e1) said infection is carried out in the presence and in the absence of a saturating dose of CCR5 co-receptor inhibitor where said indicator expresses CCR5, or (e2) said infection is carried out in the presence and in the absence of a saturating dose of CXCR4 co-receptor inhibitor where said indicator cell expresses CXCR4;
f) separately, (f1) where said indicator cell expresses CCR5, infecting at least another indicator host cell of the same type with a X4-tropic control virus in the presence and in the absence of said saturating dose of CCR5 co-receptor inhibitor,
or (f2) where said indicator cell expresses CXCR4, infecting at least another indicator cell of the same type with a R5-tropic control virus in the presence and in the absence of said saturating dose of CXCR4 co-receptor inhibitor;
g) quantifying the level of marker expressed in steps (e1) and (f1), and/or in steps (e2) and (f2); and
h) calculating the ratios

$[sample/control]_{CCR5+}$ = [level of marker expressed in step (e1) / level of marker expressed in step (f1)] in the absence of the CCR5 co-receptor inhibitor, and

$[sample/control]_{CCR5-}$ = [level of marker expressed in step (e1) / level of marker expressed in step (f1)] in the presence of the saturating dose of CCR5 co-receptor inhibitor; and/or

$[sample/control]_{CXCR4+}$ = [level of marker expressed in step (e2) / level of marker expressed in step (f2)] in the absence of the CXCR4 co-receptor inhibitor,

and

$[sample/control]_{CXCR4-}$ = [level of marker expressed in step (e2) / level of marker expressed in step (f2)] in the presence of the saturating dose of CXCR4 co-receptor inhibitor,

wherein a ratio $[sample/control]_{CCR5+}$ equal or above 1.5, while the ratio $[sample/control]_{CCR5-}$ is comprised between 0.9 and 1.1 (limits included), is indicative of the presence of HIV-1 group M viruses having tropism for CCR5 co-receptor in the biological sample from the patient, and/or
wherein a ratio $[sample/control]_{CXCR4+}$ equal or above 1.5, while the ratio $[sample/control]_{CRCR4-}$ is comprised between 0.9 and 1.1 (limits included), is indicative of the presence of HIV-1 group M viruses having tropism for CXCR4 co-receptor in the biological sample from the patient.

**7.** The method according to claim 6, wherein said pair of primers consists of:

- a forward primer comprising or consisting of the sequence SEQ ID NO:1 or SEQ ID NO:6, or a fragment thereof; and
- a reverse primer comprising or consisting of the sequence SEQ ID NO:5, or SEQ ID NO:7, or a fragment thereof.

**8.** The method according to claim 6 or 7, wherein said PCR amplification comprises a RT-PCR of said extracted nucleic acids with a pair of primers having sequences SEQ ID NO:1 and SEQ ID NO:5, followed by a nested PCR with a pair of primers having sequences SEQ ID NO:6 and SEQ ID NO:7.

**9.** The method according to any of claims 6 to 8, wherein the vector used in step (b) is a pNL4-3 plasmid deleted of all or part of the V1-V3 region of the envelope gene which contains a suitable restriction site for linearization of the plasmid.

**10.** The method according to claim 9, wherein said vector is the pNL4-3 plasmid deleted of nucleotides 6612-7249, and containing the natural *NheI* restriction site for linearization of the plasmid.

**11.** The method according to any of claims 1 to 10, wherein said producer cell is a Hela cell or a 293T cell.

**12.** The method according to any of claims 1 to 11, wherein said indicator cell is a U373-CCR5 or U373-CXCR4 cell.

**13.** The method according to any of claims 1 to 12, wherein :

- step (f) further comprises (f3) where said indicator cell expresses CCR5, infecting at least another indicator cell of the same type with a R5-tropic reference virus in the presence and in the absence of said saturating dose of CCR5 co-receptor inhibitor; or (f4) where said indicator cell expresses CXCR4, infecting at least another indicator cell of the same type with a X4-tropic reference virus in the presence and in the absence of said saturating dose of CXCR4 co-receptor inhibitor; and
- step (g) further comprises quantifying the level of marker expressed in step (f3) or (f4); and
- step (h) further comprises calculating the ratios

$[\text{reference/control}]_{CCR5+}$ = [level of marker expressed in step (f3) / level of marker expressed in step (f1)] in the absence of the CCR5 co-receptor inhibitor, and

$[\text{reference/control}]_{CCR5-}$ = [level of marker expressed in step (f3) / level of marker expressed in step (f1)] in the presence of the saturating dose of CCR5 co-receptor inhibitor; and/or

$[\text{reference/control}]_{CXCR4+}$ = [level of marker expressed in step (f4) / level of marker expressed in step (f2)] in the absence of the CXCR4 co-receptor inhibitor, and

$[\text{reference /control}]_{CXCR4-}$ = [level of marker expressed in step (f4) / level of marker expressed in step (f2)] in the presence of the saturating dose of CXCR4 co-receptor inhibitor,

wherein a ratio $[\text{reference/control}]_{CCR5+}$ equal or above 1.5, while the ratio $[\text{reference/control}]_{CCR5-}$ is comprised between 0.9 and 1.1 (limits included), indicates that all steps of the method have been successfully performed, and/or

wherein a ratio [reference/control]$_{CXCR4+}$ equal or above 1.5, while the ratio [reference/control]$_{CXCR4-}$ is comprised between 0.9 and 1.1 (limits included), ), indicates that all steps of the method have been successfully performed.

**14.** The method according to any of claims 1 to 13, wherein said R5-tropic control and/or reference virus is EnvR5 or CAR5.

**15.** The method according to any of claims 1 to 14, wherein said X4-tropic control and/or reference virus is EnvX4 or CAX4.

**16.** A method of assaying tropism of HIV-1 group M viruses present in a biological sample of a patient for CCR5 and/or CXCR4 co-receptor and susceptibility of said viruses to an entry inhibitor, which method comprises the steps consisting of:

a) subjecting nucleic acids extracted from said biological sample of a patient to a PCR amplification with a pair of primers bordering a region of HIV-1 group M genome coding for all, or essentially all, the gp120 sub-unit and for the ectodomain of gp41 sub-unit;
b) preparing a vector comprising the parts of an HIV-1 group M virus genome required for viral replication except for all or part of the region amplified in step (a);
c) transfecting a producer cell with the amplified nucleic acids of step (a) and the vector prepared in step (b) to obtain a chimeric virus by homologous recombination;
d) culturing said producer cell under conditions enabling the production of viral particles;
e) infecting an indicator cell, which is liable to be infected by an HIV-1 group M virus, which comprises a marker gene which is only activated upon viral infection and which expresses either CCR5 or CXCR4 co-receptor, with the viral particles obtained in step (d),
wherein (e1) said infection is carried out in the presence and in the absence of a saturating dose of a CCR5 co-receptor inhibitor where said indicator cell expresses CCR5, or (e2) said infection is carried out in the presence and in the absence of a saturating dose of a CXCR4 co-receptor inhibitor where said indicator cell expresses CXCR4;
f) separately, (f1) where said indicator cell expresses CCR5, infecting at least another indicator cell of the same type with a X4-tropic control virus in the presence and in the absence of said saturating dose of CCR5 co-receptor inhibitor, or (f2) where said indicator cell expresses CXCR4, infecting at least another indicator cell of the same type with a R5-tropic control virus in the presence and in the absence of said saturating dose of CXCR4 co-receptor inhibitor;
g) quantifying the level of marker expressed in steps (e1) and (f1), and/or in steps (e2) and (f2); and
h) calculating the ratios

[sample/control]$_{CCR5+}$ = [level of marker expressed in step (e1) / level of marker expressed in step (f1)] in the absence of the CCR5 co-receptor inhibitor, and

[sample/control]$_{CCR5-}$ = [level of marker expressed in step (e1) / level of marker expressed in step (f1)] in the presence of said saturating dose of CCR5 co-receptor inhibitor; and/or

[sample/control]$_{CXCR4+}$ = [level of marker expressed in step (e2) / level of marker expressed in step (f2)] in the absence of the CXCR4 co-receptor inhibitor, and

$[sample/control]_{CXCR4-}$ = [level of marker expressed in step (e2) / level of marker expressed in step (f2)] in the presence of said saturating dose of CXCR4 co-receptor inhibitor;

wherein a ratio $[sample/control]_{CCR5+}$ equal or above 1.5, while the ratio $[sample/control]_{CCR5-}$ is comprised between 0.9 and 1.1 (limits included), is indicative of the presence of HIV-1 group M viruses, in the biological sample from the patient, having tropism for CCR5 co-receptor, and/or
wherein a ratio $[sample/control]_{CXCR4+}$ equal or above 1.5, while the ratio $[sample/control]_{CXCR4-}$ is comprised between 0.9 and 1.1 (limits included), is indicative of the presence of HIV-1 group M viruses, in the biological sample from the patient, having tropism for CXCR4 co-receptor; and
i) transfecting a producer cell with the amplified nucleic acids of step (a) and the vector prepared in step (b) to obtain a chimeric virus by homologous recombination;
j) culturing said producer cell of step (j) under conditions enabling the production of viral particles;
k) incubating an indicator cell, which is liable to be infected by an HIV-1 group M virus, which comprises a marker gene which is only activated upon viral infection and which expresses CCR5, if the viruses have tropism for CCR5 co-receptor, and/or an indicator cell, which is liable to be infected by an HIV-1 group M virus, which comprises a marker gene which is only activated upon viral infection and which expresses CXCR4, if the viruses have tropism for CXCR4 co-receptor, in the absence and in the presence of an entry inhibitor;
l) infecting said indicator cells which have been incubated in the absence and in the presence of said entry inhibitor in step (k), with the viral particles obtained in step (j); and
m) quantifying the level of marker expressed by said indicator cells which have been subjected to infection in step (l);

wherein if the level of marker expressed by indicator cells which have been incubated in the presence of the entry inhibitor is decreased by comparison with the level of marker expressed by indicator cells which have been incubated in the absence of the entry inhibitor, then the viruses present in the biological sample of the patient are susceptible to said entry inhibitor.

**17.** The method according to claim 16, wherein in step (k), indicator cells are incubated in the absence and in the presence of increasing concentrations of the entry inhibitor, and step (m) further comprises determining an inhibitory concentration index of said entry inhibitor.

**18.** The method according to claim 16 or 17, wherein the producer cells of steps (c) and (i) are cells from a same cell line; and/or the indicator cells of steps (e) and (k) are cells from a same cell line.

**19.** A kit for assaying susceptibility of HIV-1 group M viruses present in a biological sample of a patient to CCR5 and/or CXCR4 co-receptor inhibitors, and/or of assaying tropism of said HIV-1 group M viruses for CCR5 and/or CXCR4 co-receptor, may comprise:

a) at least pair of primers bordering a region of HIV-1 group M genome coding for all, or essentially all, the gp120 sub-unit and for the ectodomain of gp41 sub-unit;
b) a vector comprising the parts of an HIV-1 group M virus genome required for viral replication except for all or part of the region amplified with the pair(s) of primers defined in (a);
c) a producer cell which is suitable to support the production of HIV-1 viral particles, and which is preferably not permissive for HIV-1 infection;
d) an indicator cell which is liable to be infected by an HIV-1 group M virus, which comprises a marker gene which is only activated upon viral infection and which expresses either CCR5 or CXCR4 co-receptor;
e) a R5-tropic virus and a CCR5 co-receptor inhibitor, and/or a X4-tropic virus and a CXCR4 co-receptor inhibitor;
f) optionally, reagents required to carry out PCR amplification; and
g) optionally, reagents required to quantify the level of marker expressed.

**20.** The kit according to claim 19, which comprises:

- a pair of primers having sequences SEQ ID NO:1 and SEQ ID NO:2, and
- a pair of primers having sequences SEQ ID NO:3 and SEQ ID NO:4.

**21.** The kit according to claim 19 or 20, which comprises a pNL4-3 plasmid deleted of nucleotides 6480-8263 and replaced by a linker containing the *MluI* restriction site.

**22.** A kit for assaying tropism of HIV-1 group M viruses present in a biological sample of a patient for CCR5 and/or CXCR4 co-receptor, which comprises:

a) a pair of primers bordering the entire region of HIV-1 group M genome coding for V1-V3 loops of gp120 sub-unit;
b) a vector comprising the parts of an HIV-1 group M virus genome required for viral replication except for all or part of the region amplified with the pair of primers defined in (a);
c) a producer cell which is suitable to support the production of HIV-1 viral particles, and which is preferably not permissive for HIV-1 infection;
d) an indicator cell which is liable to be infected by an HIV-1 group M virus, which comprises a marker gene which is only activated upon viral infection and which expresses either CCR5 or CXCR4 co-receptor;
e) a R5-tropic virus and a CCR5 co-receptor inhibitor, and/or a X4-tropic virus and a CXCR4 co-receptor inhibitor;
f) optionally, reagents required to carry out PCR amplification; and
g) optionally, reagents required to quantify the level of marker expressed.

**23.** The kit according to claim 22, which comprises:

- a pair of primers having sequences SEQ ID NO:1 and SEQ ID NO:5, and
- a pair of primers having sequences SEQ ID NO:6 and SEQ ID NO:7.

**24.** The kit according to claim 22 or 23, which comprises a pNL4-3 plasmid deleted of nucleotides 6612-7249, and containing the natural *NheI* restriction site for linearization of the plasmid.

```
6171  AATAGACAGG TTAATTGATA GACTAATAGA AAGAGCAGAA GACAGTGGCA
                                E00→
6221  ATGAGAGTGA AGGAGAAGTA TCAGCACTTG TGGAGATGGG GGTGGAAATG
      Env signal peptide
6271  GGGCACCATG CTCCTTGGGA TATTGATGAT CTGTAGTGCT ACAGAAAAAT
                                         gp120            E10→
6321  TGTGGGTCAC AGTCTATTAT GGGGTACCTG TGTGGAAGGA AGCAACCACC

6371  ACTCTATTTT GTGCATCAGA TGCTAAAGCA TATGATACAG AGGTACATAA

6421  TGTTTGGGCC ACACATGCCT GTGTACCCAC AGACCCCAAC CCACAAGAAG
           E20→
6471  TAGTATTGG/T AAATGTGACA GAAAATTTTA ACATGTGGAA AGATGACATG
                deletion ΔENV→
6521  GTAGAACAGA TGCATGAGGA TATAATCAGT TTATGGGATC AAAGCCTAAA

6571  GCCATGTGTA AAATTAACCC CACTCTGTGT TAGTTTAAAG TG/CACTGATT
                                                 V1/deletion ΔV→
6621  TGAAGAATGA TACTAATACC AATAGTAGTA GCGGGAGAAT GATAATGGAG

6671  AAAGGAGAGA TAAAAAACTG CTCTTTCAAT ATCAGCACAA GCATAAGAGA

6721  TAAGGTGCAG AAAGAATATG CATTCTTTTA TAAACTTGAT ATAGTACCAA

6771  TAGATAATAC CAGCTATAGG TTGATAAGTT GTAACACCTC AGTCATTACA

6821  CAGGCCTGTC CAAAGGTATC CTTTGAGCCA ATCCCCATAC ATTATTGTGC

6871  CCCGGCTGGT TTTGCGATTC TAAAATGTAA TAATAAGACG TTCAATGGAA

6921  CAGGACCATG TACAAATGTC AGCACAGTAC AATGTACACA TGGAATCAGG

6971  CCAGTAGTAT CAACTCAACT GCTGTTAAAT GGCAGTCTAG CAGAAGAAGA

7021  TGTAGTAATT AGATCTGCCA ATTTCACAGA CAATGCTAAA ACCATAATAG

7071  TACAGCTGAA CACATCTGTA GAAATTAATT GTACAAGACC CAACAACAAT
                                  V3
7121  ACAAGAAAAA GTATCCGTAT CCAGAGGGGA CCAGGGAGAG CATTTGTTAC
```

**FIG.1**

7171 AATAGGAAAA ATAGGAAATA TGAGACAAGC ACAT/TGTAAC ATTAGTAGAG
V3/C3

7221 CAAAATGGAA TGCCACTTTA AAACAGATAG/ CTAGCAAATT AAGAGAACAA
←deletion ΔV

7271 TTTGGAAATA ATAAAACAAT AATCTTTAAG CAATCCTCAG GAGGGGACCC

7321 AGAAATTGTA ACGCACAGTT TTAATTGTGG AGGGGAATTT TTCTACTGTA
←E115

7371 ATTCAACACA ACTGTTTAAT AGTACTTGGT TTAATAGTAC TTGGAGTACT

7421 GAAGGGTCAA ATAACACTGA AGGAAGTGAC ACAATCACAC TCCCATGCAG

7471 AATAAAACAA TTTATAAACA TGTGGCAGGA AGTAGGAAAA GCAATGTATG

7521 CCCCTCCCAT CAGTGGACAA ATTAGATGTT CATCAAATAT TACTGGGCTG

7571 CTATTAACAA GAGATGGTGG TAATAACAAC AATGGGTCCG AGATCTTCAG

7621 ACCTGGAGGA GGCGATATGA GGGACAATTG GAGAAGTGAA TTATATAAAT
←ES8B

7671 ATAAAGTAGT AAAAATTGAA CCATTAGGAG TAGCACCCAC CAAGGCAAAG

7721 AGAAGAGTGG TGCAGAGAGA AAAA/AGAGCA GTGGGAATAG GAGCTTTGTT
gp120/gp41

7771 CCTTGGGTTC TTGGGAGCAG CAGGAAGCAC TATGGGCGCA GCGTCAATGA

7821 CGCTGACGGT ACAGGCCAGA CAATTATTGT CTGATATAGT GCAGCAGCAG

7871 AACAATTTGC TGAGGGCTAT TGAGGCGCAA CAGCATCTGT TGCAACTCAC

7921 AGTCTGGGGC ATCAAACAGC TCCAGGCAAG AATCCTGGCT GTGGAAAGAT

7971 ACCTAAAGGA TCAACAGCTC CTGGGGATTT GGGGTTGCTC TGGAAAACTC

8021 ATTTGCACCA CTGCTGTGCC TTGGAATGCT AGTTGGAGTA ATAAATCTCT

8071 GGAACAGATT TGGAATAACA TGACCTGGAT GGAGTGGGAC AGAGAAATTA

8121 ACAATTACAC AAGCTTAATA CACTCCTTAA TTGAAGAATC GCAAAACCAG

FIG.2

8171 CAAGAAAAGA ATGAACAAGA ATTATTGGAA TTAGATAAAT GGGCAAGTTT

8221 GTGGAATTGG TTTAACATAA CAAATTGGCT GTGGTATATA AAA/TTATTCA
←deletion ΔENV/MS domain→

8271 TAATGATAGT AGGAGGCTTG GTAGGTTTAA GAATAGTTTT TGCTGTACTT

8321 TCTATAGTG/A ATAGAGTTAG GCAGGGATAT TCACCATTAT CGTTTCAGAC
←MS domain/cytoplasmic domain→

8371 CCACCTCCCA ATCCCGAGGG GACCCGACAG GCCCGAAGGA ATAGAAGAAG

8421 AAGGTGGAGA GAGAGGCAGA GACAGATCCA TTCGATTAGT GAACGGATCC

8471 TTAGCACTTA TCTGGGACGA TCTGCGGAGC CTGTGCCTCT TCAGCTACCA
←FuB

8521 CCGCTTGAGA GACTTACTCT TGATTGTAAC GAGGATTGTG GAACTTCTGG
←FuB

8571 GACGCAGGGG GTGGGAAGCC CTCAAATATT GGTGGAATCT CCTACAGTAT

8621 TGGAGTCAGG AACTAAAGAA TAGTGCTGTT AACTTGCTCA ATGCCACAGC

8671 CATAGCAGTA GCTGAGGGGA CAGATAGGGT TATAGAAGTA TTACAAGCAG

8721 CTTATAGAGC TATTCGCCAC ATACCTAGAA GAATAAGACA GGGCTTGGAA

8771 AGGATTTTGC TATAAGATGG GTGGCAAGTG GTCAAAAAGT AGTGTGATTG

8821 GATGGCCTGC TGTAAGGGAA AGAATGAGAC GAGCTGAGCC AGCAGCAGAT

8871 GGGGTGGGAG CAGTATCTCG AGACCTAGAA AAACATGGAG CAATCACAAG

8921 TAGCAATACA GCAGCTAACA ATGCTGCTTG TGCCTGGCTA GAAGCACAAG

8971 AGGAGGAAGA GGTGGGTTTT CCAGTCACAC CTCAGGTACC TTTAAGACCA

9021 ATGACTTACA AGGCAGCTGT AGATCTTAGC CACTTTTTAA AAGAAAAGGG

9071 GGGACTGGAA GGGCTAATTC
←E01-

FIG.3

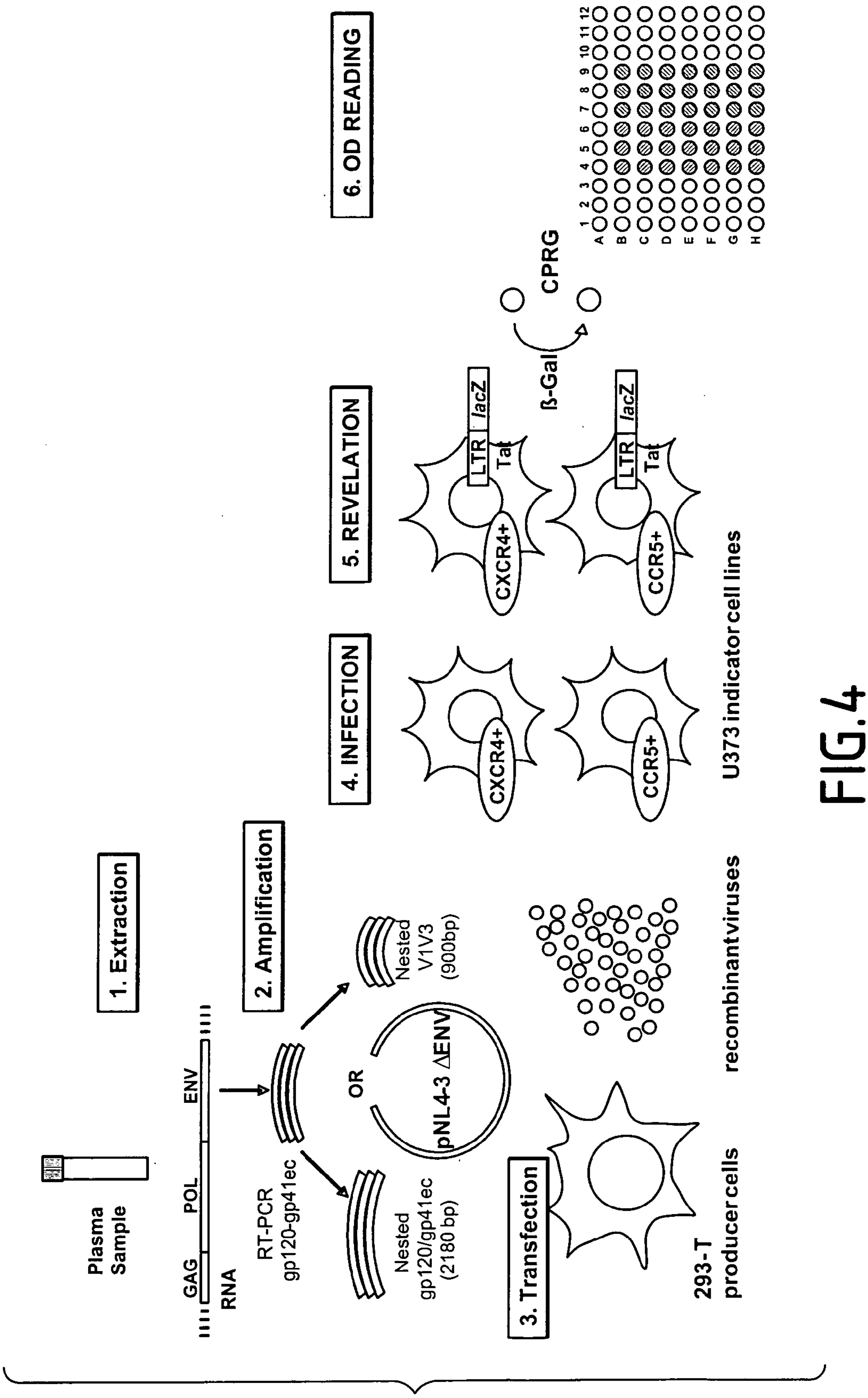
1. Extraction
Plasma Sample
GAG
POL
ENV
RNA
2. Amplification
RT-PCR
gp120-gp41ec
Nested
gp120/gp41ec
(2180 bp)
OR
Nested
V1V3
(900bp)
pNL4-3 ΔENV
3. Transfection
293-T
producer cells
recombinant viruses
4. INFECTION
CXCR4+
CCR5+
U373 indicator cell lines
5. REVELATION
CXCR4+
LTR
lacZ
Tat
CCR5+
LTR
lacZ
Tat
ß-Gal
CPRG
6. OD READING
1 2 3 4 5 6 7 8 9 10 11 12
A
B
C
D
E
F
G
H

FIG.4

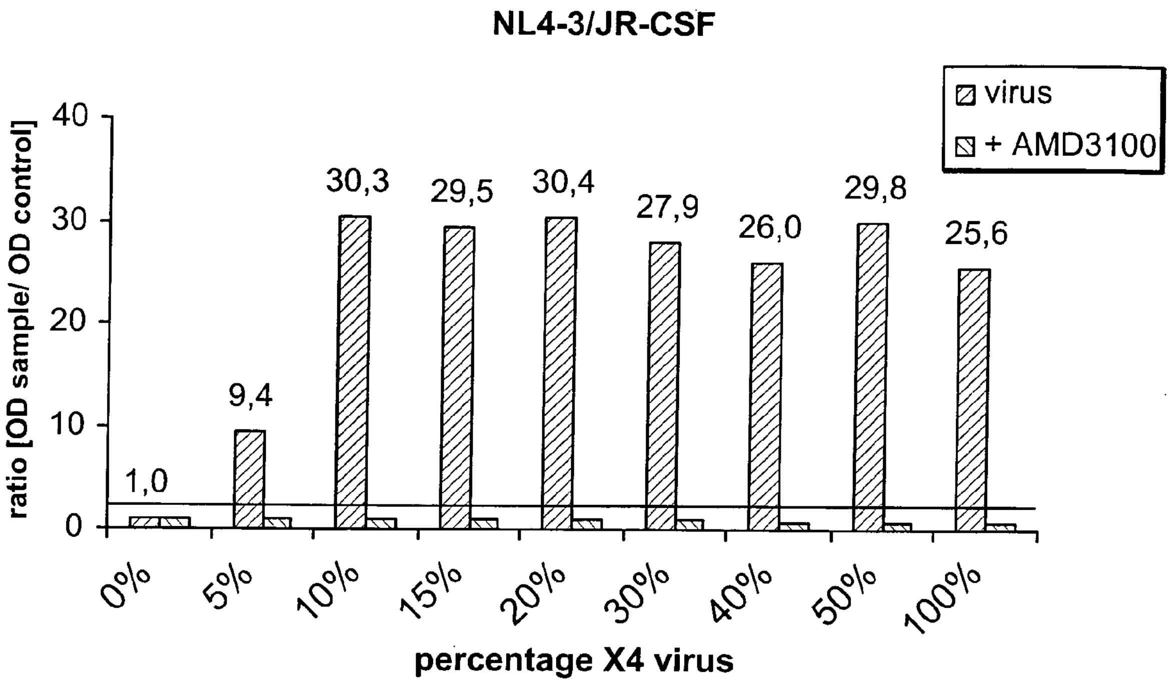
NL4-3/JR-CSF
virus
+ AMD3100
ratio [OD sample/ OD control]
40
30
20
10
0
1,0
9,4
30,3
29,5
30,4
27,9
26,0
29,8
25,6
0%
5%
10%
15%
20%
30%
40%
50%
100%
percentage X4 virus

FIG.5

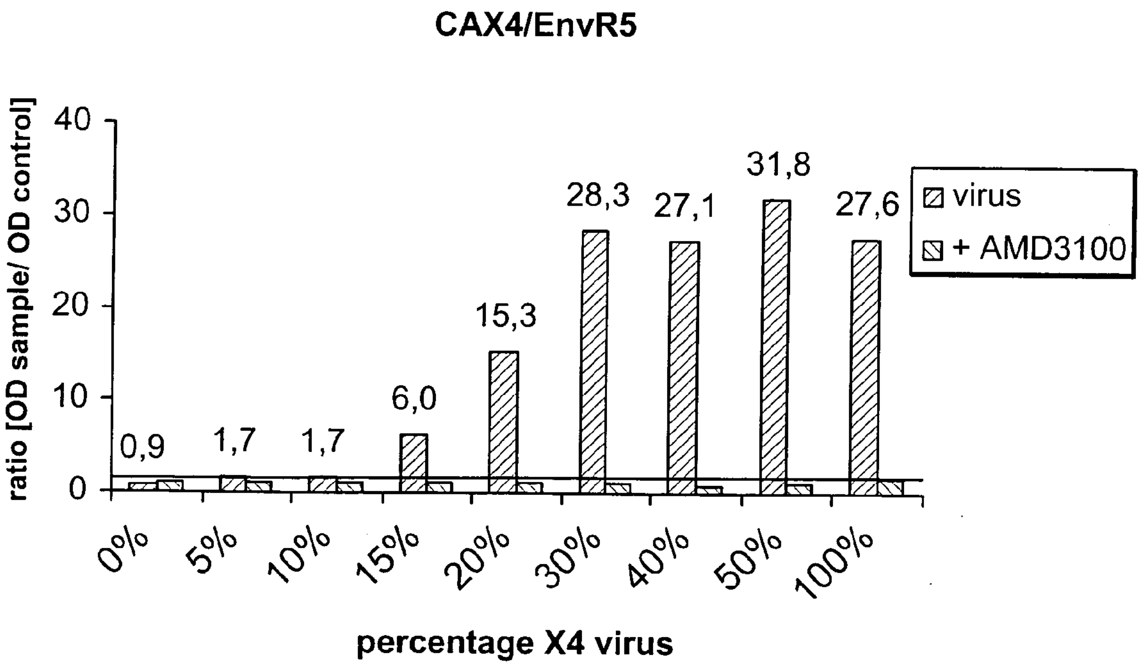
CAX4/EnvR5
virus
+ AMD3100
ratio [OD sample/ OD control]
40
30
20
10
0
0,9
1,7
1,7
6,0
15,3
28,3
27,1
31,8
27,6
0%
5%
10%
15%
20%
30%
40%
50%
100%
percentage X4 virus

FIG.6

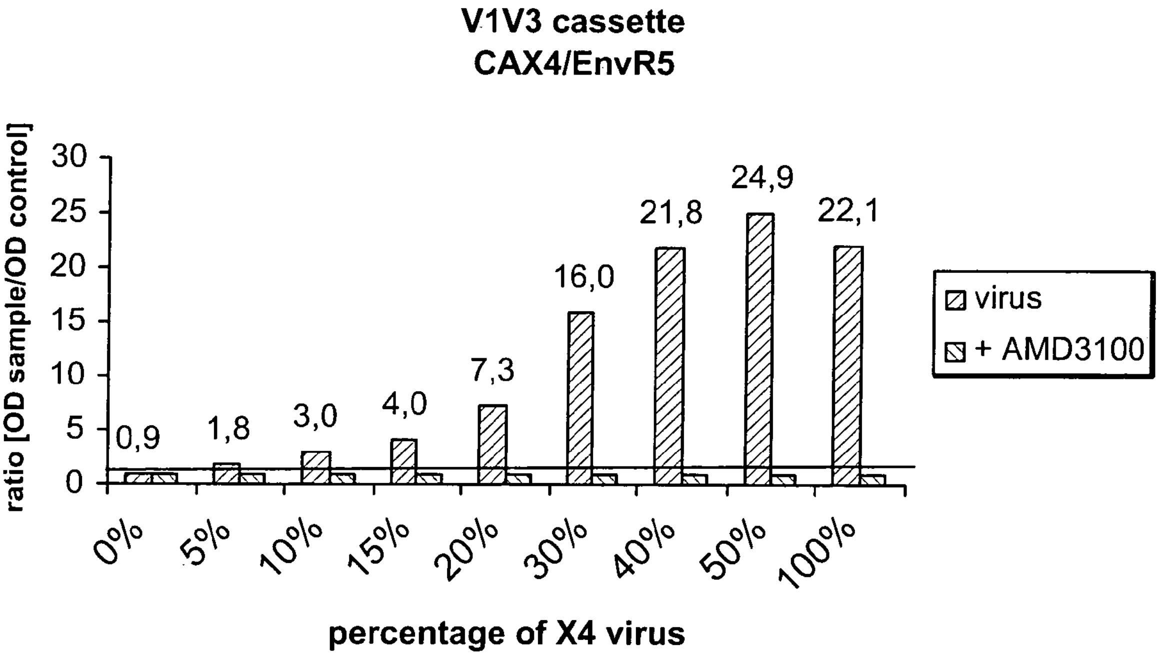
V1V3 cassette
CAX4/EnvR5
ratio [OD sample/OD control]
30
25
20
15
10
5
0
0,9
1,8
3,0
4,0
7,3
16,0
21,8
24,9
22,1
0%
5%
10%
15%
20%
30%
40%
50%
100%
percentage of X4 virus
virus
+ AMD3100

FIG.7

European Patent Office

# EUROPEAN SEARCH REPORT

Application Number
EP 06 29 1637

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | WO 02/38792 A (BIOALLIANCE PHARMA [FR]; INST NAT SANTE RECH MED [FR]; CLAVEL FRANCOIS) 16 May 2002 (2002-05-16) see whole doc. esp. claims 18,19,20 ----- | 1-24 | INV. C12N15/10 C12N15/86 |
| D,X | WO 02/27321 A2 (HEALTH RESEARCH INC [US]) 4 April 2002 (2002-04-04) see whole doc. esp. clamis ----- | 1-24 | |
| D,X | WO 03/070985 A (VIROLOGIC INC [US]; RICHMAN DOUGLAS [US]; WRIN MARY T [US]; LITTLE SUS) 28 August 2003 (2003-08-28) see whole doc. esp. claims and expl.9, p.54 and p.55,l.10 ff. ----- | 1-24 | |
| A | SCARLATTI G ET AL: "IN VIVO EVOLUTION OF HIV-1 CO-RECEPTOR USAGE AND SENSITIVITY TO CHEMOKINE-MEDIATED SUPPRESSION" NATURE MEDICINE, NATURE AMERICA, NEW YORK, US, vol. 3, no. 11, November 1997 (1997-11), pages 1259-1265, XP001024255 ISSN: 1078-8956 * the whole document * ----- | | TECHNICAL FIELDS SEARCHED (IPC) C12N |
| A | WILLEY ET AL: "Amino acid substitutions in the human immunodeficiency virus type 1 gp120 V3 loop that change viral tropism also alter physical and functional properties of the virion envelope" JOURNAL OF VIROLOGY, THE AMERICAN SOCIETY FOR MICROBIOLOGY, US, vol. 68, no. 7, July 1994 (1994-07), pages 4409-4419, XP002119922 ISSN: 0022-538X * the whole document * ----- | | |

The present search report has been drawn up for all claims

2

| Place of search | Date of completion of the search | Examiner |
|---|---|---|
| Munich | 23 February 2007 | Mueller, Frank |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 06 29 1637

This annex lists the patent family members relating to the patent documents cited in the above-mentioned European search report.
The members are as contained in the European Patent Office EDP file on
The European Patent Office is in no way liable for these particulars which are merely given for the purpose of information.

23-02-2007

| Patent document cited in search report | Publication date | Patent family member(s) | Publication date |
|---|---|---|---|
| WO 0238792 A | 16-05-2002 | AU 2305202 A | 21-05-2002 |
| | | CA 2429073 A1 | 16-05-2002 |
| | | EP 1364071 A2 | 26-11-2003 |
| | | JP 2005500003 T | 06-01-2005 |
| | | US 2003207294 A1 | 06-11-2003 |
| | | US 2004053219 A1 | 18-03-2004 |
| WO 0227321 A2 | 04-04-2002 | AU 9122501 A | 08-04-2002 |
| | | EP 1350100 A2 | 08-10-2003 |
| WO 03070985 A | 28-08-2003 | AU 2003217399 A1 | 09-09-2003 |
| | | CA 2476365 A1 | 28-08-2003 |
| | | CN 1646706 A | 27-07-2005 |
| | | EP 1481098 A1 | 01-12-2004 |
| | | JP 2006506944 T | 02-03-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0227321 A
- WO 0238792 A
- WO 03070985 A
- GB 427857 A


### Non-patent literature cited in the description


- **KOOTSTRA ; SCHUITEMAKER.** *Methods Mol Biol,* 2005, vol. 304, 317-25
- **PETERS et al.** *J. Virol.,* 2006, vol. 80 (3), 6324-6332
- **GRAY et al.** *Virology,* 2005, vol. 337 (2), 384-98
- **KULKARNI et al.** *Virology,* 2005, vol. 337 (1), 68-75
- **A. LOW ; K. SKRABAL ; W. DONG ; F. MAMMANO ; T. SING ; P. CHEUNG ; P.R. HARRIGAN.** Is there a Gold Standard for Determining HIV Coreceptor Usage in Clinical Samples? A Comparison of two Phenotypic Tropism Assays and a Bioinformatic Model. *13th Conference on Retroviruses and Opportunistic Infections,* 05 February 2006
- **ADACHI et al.** *J. Virol.,* 1986, vol. 59 (2), 284-291
- Molecular Cloning, A Laboratory Manual. Cold Spring Harbor Laboratory Press
- **ALKHATIB et al.** *Science,* 1996, vol. 272, 1955-1958
- **SIMMONS et al.** *Science,* 1997, vol. 276, 276-279
- **HARTLEY et al.** *Proc Natl Acad Sci U S A,* 2004, vol. 101, 16460-16465
- **MENTEN et al.** *J Clin Invest,* 1999, vol. 104, R1-R5
- **PALANI et al.** *J Med Chem.,* 2001, vol. 44 (21), 3339-42
- **KAZMIERSKI et al.** *Chemical Biology & Drug Design,* 2006, vol. 67, 13
- **ICHIYAMA et al.** *Proc Nat Acad Sci U S A,* 2003, vol. 100, 4185-4190
- **DE CLERCQ.** *Nat Rev Drug Discov,* 2003, vol. 2, 581-587
- **LABROSSE et al.** *J Virol,* 1998, vol. 72 (8), 6381-6388
- **CHARNEAU et al.** *J. Mol. Biol.,* 1994, vol. 241, 651-662
- **AMARA et al.** *J. Exp. Med.,* 1997, vol. 186, 139-146
- **SKRABAL et al.** *J. Virol,* 2005, vol. 79 (18), 11848-11857
- **SKRABAL et al.** *J. Virol.,* 2005, vol. 79 (18), 11848-11857

SEQUENCE LISTING

<110> Eurofins Viralliance Inc.
INSERM

<120> Method of assaying HIV-1 tropism for CCR5 and/or CXCR4 co-receptor and or susceptibility to CCR5 and/or CXCR4 co-receptor inhibitors

<130> BET 06P1024

<160> 7

<170> PatentIn version 3.3

<210> 1
<211> 28
<212> DNA
<213> Artificial

<220>
<223> primer

<400> 1
tagaaagagc agaagacagt ggcaatga 28

<210> 2
<211> 26
<212> DNA
<213> Artificial

<220>
<223> primer

<400> 2
tccagtcccc ccttttcttt taaaaa 26

<210> 3
<211> 26
<212> DNA
<213> Artificial

<220>
<223> primer

<400> 3
ttgtgggtca cagtctatta tggggt 26

<210> 4
<211> 23
<212> DNA
<213> Artificial

<220>
<223> primer

<400> 4
ggtggtagct gaagaggcac agg 23

```
<210>  5
<211>  21
<212>  DNA
<213>  Artificial

<220>
<223>  primer

<400>  5
cacttctcca attgtccctc a                                        21


<210>  6
<211>  27
<212>  DNA
<213>  Artificial

<220>
<223>  primer

<400>  6
gggccacaca tgcctgtgta cccacag                                  27


<210>  7
<211>  24
<212>  DNA
<213>  Artificial

<220>
<223>  primer

<400>  7
agaaaaattc cctccacaa ttaa                                      24
```